# EUROPEAN PATENT APPLICATION

(11) **EP 4 190 346 A1**
(43) Date of publication of application: **07.06.2023**
(21) Application number: 21850159.1
(22) Date of filing: 02.07.2021
(51) Int. Cl.: A61K 38/16, A61P 29/00, A61P 19/00, G01N 33/68

(54) **COMPOSITION FOR PREVENTING OR TREATING INFLAMMATORY DISEASES, AND USE THEREOF**

(30) Priority: 31.07.2020 KR 20200095892
(71) Applicant: Seoul National University Hospital, Seoul 03080 (KR)
(72) Inventor: JO, Hyun Chul, Seoul 05502 (KR); LEE, Ah-Young, Anyang-si Gyeonggi-do 14043 (KR); YEA, Ji-Hye, Hwaseong-si Gyeonggi-do 18530 (KR)
(74) Representative: V.O.
(86) International application number: PCT/KR2021/008402
(87) International publication number: WO 2022/025455

(57) **Abstract**

The present disclosure relates to a composition for preventing or treating an inflammatory disease. Zkscan8, which is presented in the present disclosure, has a superior effect of effectively inhibiting inflammatory responses by inhibiting the expression of inflammatory cytokines and promoting the expression of anti-inflammatory factors. Therefore, it is applicable to a composition for preventing or treating an inflammatory disease.

## Description

### [Technical Field]

The present disclosure relates to a Zkscan8 gene related with inflammatory diseases and a composition for preventing or treating an inflammatory disease, which contains the same.

### [Background Art]

Inflammatory disease refers to a condition where swelling, redness and sharp pain occur together with infiltration of inflammatory immune cells into various organs or tissues of the human body and histological changes are accompanied due to various external stimuli or endogenous factors. The inflammation is triggered by various chemical mediators produced by damaged tissues and migrating cells. A variety of chemical mediators are known depending on the type of inflammatory processes. Normally, the body neutralizes or removes pathogens through inflammatory responses and regenerate the damaged tissues to recover their normal structures and functions. But sometimes, the situation worsens to disease states such as chronic inflammation.

The most common drugs for preventing or treating inflammatory diseases are classified largely into steroidal and non-steroidal drugs. Because most of the synthetic drugs for preventing or treating inflammatory diseases have many side effects in addition to their principal action, the development of an agent for preventing or treating inflammatory diseases, which has excellent efficacy and has few side effects, is desperately needed.

In order to solve these problems, mesenchymal stem cells (MSCs) are emerging as a new option for treating inflammatory diseases. However, the development of a MSC therapeutic agent optimized to have strong effect on inflammatory diseases is still unsatisfactory.

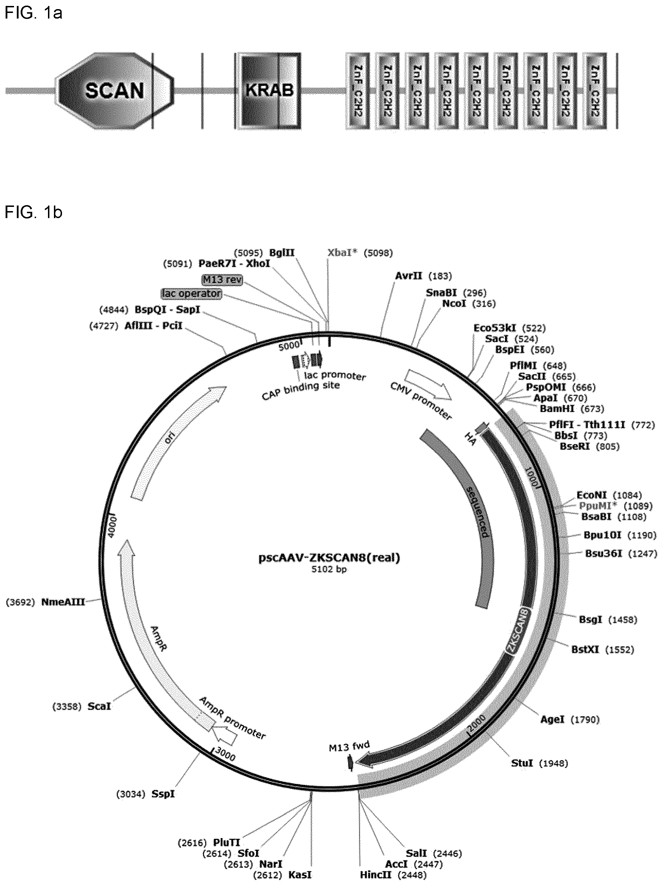

Zkscan8 (zinc finger protein with KRAB and SCAN domains 8) is a class of C2H2-ZNF (zinc finger) protein transcription factor. It is a transcription factor having a KRAB (Kruppel-associated box) domain and SRE-ZBP and SCAN (CTfin51, AW-1 and Number 18 cDNA) domains towards the N-terminal (FIG. 1a).

ZFPs (zinc finger proteins) are reported to have various biological activities. In particular, it is known to be expressed in various early progenitor cells including embryonic stem cells (ESCs). It is known that ZNF 589, 268 and 300 affect the differentiation of hematopoietic stem cells. However, the biological characteristics of Zkscan8 (ZNF 192, LD5-1, Zscan 40) have not been reported yet. Particularly, the function of Zkscan8 in inflammatory diseases has not been researched yet.

The inventors of the present disclosure have identified the potential of Zkscan8 in treatment of inflammatory disease, and have completed the present disclosure by identifying its effect on the prevention and treatment of inflammatory disease.

Throughout the present specification, a number of papers and patent documents are referred to and their citations are indicated. The disclosed contents of the cited papers and patent documents are included herein by reference in their entirety, so that the level of the related art and the contents of the present disclosure can be described more clearly.

### [References of Related Art]

### [Patent Documents]

(Patent document 1) Korean Patent Registration No. 10-1067816.

### [Disclosure]

### [Technical Problem]

The inventors of the present disclosure have made consistent efforts to develop a method for effectively preventing or treating inflammatory diseases. As a result, they have completed the present disclosure by finding out that inflammatory diseases can be prevented, ameliorated or treated by overexpressing Zkscan8 (zinc finger protein with KRAB and SCAN domains 8).

The present disclosure is directed to providing a composition for preventing or treating an inflammatory disease.

The present disclosure is also directed to providing a method for screening a composition for preventing or treating an inflammatory disease.

The present disclosure is also directed to providing a novel use of a Zkscan8 (zinc finger protein with KRAB and SCAN domains 8) gene for preparation of a drug or a veterinary drug for treating an inflammatory disease.

Other purposes and advantages of the present disclosure become more apparent by the following detailed description, claims and drawings.

### [Technical Solution]

In an aspect of the present disclosure, the present disclosure provides a composition for preventing or treating an inflammatory disease, which contains a Zkscan8 (zinc finger protein with KRAB and SCAN domains 8) protein as an active ingredient.

The inventors of the present disclosure have made consistent efforts to develop an effective therapeutic agent composition having superior therapeutic activity for inflammatory diseases. As a result, they have found out that, in cells in which inflammation is induced by IL-1β, IL-10 can be activated or inflammation can be alleviated, prevented or treated by lowering the expression level of inflammatory response-related genes (COX-2, mPGES-1, TNF-α, MMP-1, MMP-3), and it can be effectively used for treatment of inflammatory diseases.

In a specific exemplary embodiment of the present disclosure, Zkscan8 (zinc finger protein with KRAB and SCAN domains 8) is a class of C2H2-ZNF (zinc finger) protein transcription factor. It is a transcription factor having a KRAB (Kruppel-associated box) domain and SRE-ZBP and SCAN (CTfin51, AW-1 and Number 18 cDNA) domains towards the N-terminal.

According to the present disclosure, the Zkscan8 protein encoded by the Zkscan8 gene may have an amino acid sequence registered with NCBI accession number NP_001265048 and may be represented by SEQ ID NO 2.

In the present disclosure, the Zkscan8 protein may include a variant or a functional equivalent thereof. The variant or functional equivalent of the protein refers to a polypeptide which has a sequence homology of 60%, specifically 70%, more specifically 80%, or higher to the amino acid sequence of the Zkscan8 protein as a result of the addition, substitution or deletion of amino acids, and exhibits substantially the same activity as the Zkscan8 protein of the present disclosure. The functional equivalent may include, for example, an amino acid sequence variant with some of the amino acids in the amino acid sequence of the Zkscan8 protein being substituted, deleted or added. The substitution of amino acids may be specifically conservative substitution, and examples of naturally occurring conservative substitution of amino acids include: aliphatic amino acids (Gly, Ala, Pro), hydrophobic amino acids (Ile, Leu, Val), aromatic amino acids (Phe, Tyr, Trp), acidic amino acids (Asp, Glu), basic amino acids (His, Lys, Arg, Gln, Asn) and sulfur-containing amino acids (Cys, Met). The deletion of amino acids may specifically at the regions not directly involved in the activity of the Zkscan8 protein of the present disclosure. In addition, a polypeptide derivative whose chemical structure has been changed partially while retaining the basic structure and physiological activity of the Zkscan8 protein may be encompassed in the scope of the functional equivalent. For example, a fusion protein, etc. prepared from fusion with another protein in order to change the stability, storage property, volatility, solubility, etc. of the Zkscan8 protein of the present disclosure while retaining the basic structure and physiological activity may be included.

The Zkscan8 protein according to the present disclosure may be used as it is or in the form of a pharmaceutically acceptable salt. In the present disclosure, "pharmaceutically acceptable" means that the protein is physiologically and does not normally cause allergic reactions such as gastrointestinal disorder or dizziness or similar responses when administered to human without inhibiting the action of the active ingredient. The salt may be specifically an acid addition salt formed from a pharmaceutically acceptable free acid. As the free acid, an organic acid or an inorganic acid. The organic acid includes but is not limited to citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, glutamic acid and aspartic acid. And, the inorganic acid includes but is not limited to hydrochloric acid, bromic acid, sulfuric acid and phosphoric acid.

In the present disclosure, the Zkscan8 protein or a fragment thereof may be extracted from the nature, synthesized (Merrifield, J. Amer. chem. Soc. 85: 2149-2156, 1963) or prepared by gene recombination based on a DNA sequence (Sambrook et al, Molecular Cloning, Cold Spring Harbor Laboratory Press, New York, USA, 2nd edition, 1989).

In the present disclosure, the term "inflammatory disease" collectively refers to a disease having inflammation as a main lesion Specifically, the inflammatory disease may be one or more selected from a group consisting of dermatitis, allergy, atopy, asthma, bronchitis, conjunctivitis, periodontitis, rhinitis, otitis media, sore throat, tonsillitis, pneumonia, gastric ulcer, gastritis, Crohn's disease, colitis, peritonitis, osteomyelitis, meningitis, encephalitis, cystic fibrosis, stroke, hemorrhoids, gout, ankylosing spondylitis, spondyloarthropathy, enteropathy, spondylitis, rheumatic fever, lupus, fibromyalgia, psoriatic arthritis, osteoarthritis, rheumatoid arthritis, frozen shoulder, tendinitis, tenosynovitis, peritendinitis, tenosynovitis, myositis, hepatitis, cystitis, nephritis, Sjogren's syndrome, multiple sclerosis and acute and chronic inflammatory diseases. More specifically, the inflammatory disease may be one or more selected from a group consisting of psoriatic arthritis, osteoarthritis, rheumatoid arthritis, frozen shoulder, tendinitis, tenosynovitis, peritendinitis, tenosynovitis and myositis.

In the present specification, the term "prevention" refers to prevention of the occurrence of a disease or a disorder in a subject who has not been diagnosed with the disease or disorder but has the possibility of having the disease or disorder.

In the present specification, the term "treatment" refers to: (a) inhibition of the development of a disease, a disorder or symptoms; (b) alleviation of disease, a disorder or symptoms; or (c) removal of a disease, a disorder or symptoms. When the composition of the present disclosure is administered to a subject, it inhibits the development of the symptoms of a musculoskeletal disease or removes or alleviates them by promoting or activating the expression of Zkscan8, thereby promoting differentiation into the musculoskeletal system and inducing recovery of musculoskeletal tissues and formation of collagen. Accordingly, the composition of the present disclosure may be used as a composition for treating a musculoskeletal disease on its own or may be used as a therapeutic adjuvant for the disease as being administered together with another pharmacological ingredient. Accordingly, in the present specification, the term "treatment" or "therapeutic agent" encompasses "therapeutic support" or "therapeutic adjuvant".

In the present specification, the term "administration" or "administer" refers to administration of a therapeutically effective amount of the composition of the present disclosure directly into a subject.

In the present disclosure, the term "therapeutically effective amount" refers to the content of the composition of the present disclosure containing the active ingredient in an amount enough to provide a therapeutic or prophylactic effect in a subject. Therefore, the term includes the meaning of "prophylactically effective amount".

In the present specification, the term "subject" includes human, mouse, rat, guinea pig, dog, cat, horse, cow, pig, monkey, chimpanzee, baboon or rhesus monkey without limitation. Specifically, the subject of the present disclosure is human.

The Zkscan8 (zinc finger protein with KRAB and SCAN domains 8) of the present disclosure protein is effective in treating and preventing inflammatory diseases. Specifically, it inhibits the expression of the Cox-2, mPGES-1 and TNF-α genes which are direct mediators of inflammatory responses, promotes the expression of IL-10 (interluekin-10) and inhibits the expression of MMP-1 and MMP-3.

In an example of the present disclosure, it was confirmed that, when a tendinitis-induced rat is treated with Zkscan8-overexpressed cells, the inflammatory response of tendon tissue is suppressed and recovery is promoted as compared to an untreated rat as the expression of the Cox-2, mPGES-1 and TNF-α genes, which are direct mediators of inflammatory responses, is inhibited, the expression of IL-10 (interluekin-10) is promoted and the expression of MMP-1 and MMP-3 is inhibited. Accordingly, a composition containing the Zkscan8 protein can exhibit an effect of preventing or treating inflammatory diseases.

In another aspect of the present disclosure, the present disclosure provides a pharmaceutical composition for preventing and treating an inflammatory disease, which contains the Zkscan8 (zinc finger protein with KRAB and SCAN domains 8) gene, a vector including the gene, a cell including the vector or a culture thereof as an active ingredient.

The description about the inflammatory disease to be diagnosed or prevented or treated in the present disclosure will be omitted to avoid unnecessary redundancy because it was described in detail above.

The present disclosure may relate to a novel use of the Zkscan8 gene for preventing or treating an inflammatory disease.

In a specific exemplary embodiment of the present disclosure, Zkscan8 (zinc finger protein with KRAB and SCAN domains 8) is a class of C2H2-ZNF (zinc finger) protein transcription factor. It is a transcription factor having a KRAB (Kruppel-associated box) domain and SRE-ZBP and SCAN (CTfin51, AW-1 and Number 18 cDNA) domains towards the N-terminal.

The Zkscan8 (zinc finger protein with KRAB and SCAN domains 8) gene may be represented by SEQ ID NO 1, which may be a base sequence registered in the NBCI with a gene symbol of Zkscan8 and a gene ID of 7745.

In an example of the present disclosure, it was confirmed that, when a tendinitis-induced rat is treated with Zkscan8-overexpressed cells, the inflammatory response of tendon tissue is suppressed and recovery is promoted as compared to an untreated rat as the expression of the Cox-2, mPGES-1 and TNF-α genes, which are direct mediators of inflammatory responses, is inhibited, the expression of IL-10 (interluekin-10) is promoted and the expression of MMP-1 and MMP-3 is inhibited. Accordingly, a composition containing the Zkscan8 (zinc finger protein with KRAB and SCAN domains 8) gene, a vector including the gene, a cell including the vector or a culture thereof as an active ingredient can exhibit an effect of preventing or treating inflammatory diseases.

In the present disclosure, the vector may be one or more selected from a group consisting of a linear DNA, a plasmid DNA and a recombinant viral vector, and the virus may be one or more selected from a group consisting of retrovirus, adenovirus, adeno-associated virus, herpes simplex virus and lentivirus.

Methods for delivering the vector of the present disclosure into a host cell includes, for example, microinjection (Harland and Weintraub, J. Cell Biol. 101: 1094-1099 (1985)), calcium phosphate precipitation (Chen and Okayama, Mol. Cell. Biol. 7: 2745-2752 (1987)), electroporation (Tur-Kaspa et al., Mol. Cell Biol., 6: 716-718 (1986)), liposome-mediated transfection (Nicolau et al., Methods Enzymol., 149: 157-176 (1987)), DEAE-dextran treatment (Gopal, Mol. Cell Biol., 5: 1188-1190 (1985)) and gene bombardment (Yang et al., Proc. Natl. Acad. Sci., 87: 9568-9572 (1990)), although not being limited thereto.

The cell including the vector may be one or more selected from a group consisting of a stem cell, a dendritic cell, an autologous tumor cell and an established tumor cell, although not being specially limited thereto.

In the present disclosure, the term "stem cell" refers to an undifferentiated cell having the ability to differentiate into various body tissues. The stem cells can be classified into totipotent stem cells, pluripotent stem cells, multipotent stem cells, etc. The term stem cell may be used interchangeably with such terms as precursor cell, progenitor cell, etc. In the present disclosure, the stem cell may be an embryonic stem cell (ESC), an induced pluripotent stem cell (iPSC) or a mesenchymal stem cell (MSC), more specifically a mesenchymal stem cell.

The mesenchymal stem cell may be a mesenchymal stem cell derived from the bone marrow, fat, umbilical cord or umbilical cord blood. Specifically, the mesenchymal stem cell may be an umbilical cord-derived mesenchymal stem cell. It is because the umbilical cord-derived mesenchymal stem cells exhibit few immune rejections and are favorable for grafting because they have less MHC class type I and type II antigens.

In an example of the present disclosure, an expression vector was prepared by inserting the Zkscan8 (zinc finger protein with KRAB and SCAN domains 8) gene of SEQ ID NO 1 into a pscAAV vector. It was named 'pscAAV-Zkscan8' and its cleavage map is shown in FIG. 1b. The prepared expression vector was transfected into human umbilical cord-derived mesenchymal stem cells (UCMSCs) (see Example 2). It was confirmed that treatment with the Zkscan8-overexpressed umbilical cord-derived mesenchymal stem cells resulted in decreased tendon thickness, swelling, redness and inflammation. In addition, the treatment with the Zkscan8-overexpressed umbilical cord-derived mesenchymal stem cells exhibited the effect of suppressing the inflammation of tendon tissue, nonspecific vascularization and the infiltration of inflammatory cells. That is to say, it can be seen that, when the Zkscan8-overexpressed umbilical cord-derived mesenchymal stem cells were applied to the inflammation of the rotator cuff, they inhibit the inflammatory response of tendon and promote the regeneration of the tendon. Accordingly, the composition of the present disclosure for preventing or treating an inflammatory disease increases the expression or activity of Zkscan8.

When the composition of the present disclosure is prepared as a pharmaceutical composition, the pharmaceutical composition of the present disclosure contains a pharmaceutically acceptable carrier.

The pharmaceutically acceptable carrier contained in the pharmaceutical composition of the present disclosure includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, etc. as those commonly used for preparation, although not being limited thereto. The pharmaceutical composition of the present disclosure may further contain, in addition to the above-described ingredients, a lubricant, a wetting agent, a sweetener, a flavorant, an emulsifier, a suspending agent, a preservative, etc. Suitable pharmaceutically acceptable carriers and preparations are described in detail in Remington's Pharmaceutical Sciences (19th ed., 1995).

The pharmaceutical composition of the present disclosure may be administered orally or parenterally. Specifically, it may be administered orally, intravenously, subcutaneously or intraperitoneally.

The administration dosage of the pharmaceutical composition of the present disclosure may be determined in consideration of various factors such as formulation method, administration method, the age, body weight, sex and pathological condition of a patient, diet, administration time, administration route, excretion rate and response sensitivity. A preferred administration dosage of the pharmaceutical composition of the present disclosure for an adult is within 0.001-100 mg /kg.

The pharmaceutical composition of the present disclosure may be formulated according to a method that can be easily employed by those having ordinary knowledge in the art to which the present disclosure belongs into a single-dose or multi-dose form by using a pharmaceutically acceptable carrier and/or excipient. The formulation may be in the form of a solution in an oily or aqueous medium, a suspension, a syrup, an emulsion, an extract, a dust, a powder, a granule, a tablet or a capsule, and may further contain a dispersant or a stabilizer.

In another aspect of the present disclosure, the present disclosure provides a method for screening a composition for preventing or treating an inflammatory disease, which includes:
(a) a step of contacting cells treated with a stimulation that can induce an inflammatory response with a test substance; and
(b) a step of measuring the expression level of Zkscan8 (zinc finger protein with KRAB and SCAN domains 8) in the sample.

If the expression level of the Zkscan8 is increased as compared to a normal control group in the step (b), the test substance is determined as a composition for preventing or treating an inflammatory disease.

The description about the inflammatory disease to be diagnosed or prevented or treated in the present disclosure will be omitted to avoid unnecessary redundancy because it was described in detail above.

In the present disclosure, the term "cell" refers to any sample obtained from a mammal including human. The cell includes a tissue, an organ, a cell or a cell culture. More specifically, the cell may be one or more selected from a group consisting of a tendon cell, a ligament cell, a muscle cell, a cartilage cell and a bone cell. Specifically, it may be a tendon cell or a ligament cell.

In the present disclosure, the term "inflammatory response" refers to a complex biological response of tissues to harmful stimuli such as pathogens, irritants, damaged cells, etc. It is a protective response involving immune cells, blood vessels and molecular mediators. The function of the inflammatory response is to eliminate the initial cause of cell injury, clear out damaged cells or tissues, and initiate tissue repair. The inflammatory response is accompanied by such symptoms as heat, pain, redness, swelling, etc.

In the present disclosure, the "stimulation that can induce an inflammatory response" refers to a stimulation that can induce an inflammatory response, specifically a stimulation that can induce a NF-kB-mediated inflammatory response, although not being specially limited thereto. The stimulation that can induce an inflammatory response may be one that can activate, trigger or amplify the NF-kB pathway, specifically one or more selected from a group consisting of TNFα (tumor necrosis factor α), IL-1β (interleukin 1-beta), PAMP (pathogen-associated molecular pattern) and LPS (lipopolysaccharide).

The term "test substance" used with reference to the screening method of the present disclosure refers to an unknown substance added to a sample containing cells expressing Zkscan8 in order to test if it affects the expression level of Zkscan8. The test substance includes a compound, a nucleotide, a peptide and a natural extract, although not being limited thereto. The step of measuring the expression level of Zkscan8 in the biological sample treated with the test substance may be performed by various expression level measurement methods known in the art. If the expression level of Zkscan8 is increased, the test substance may be determined as a composition for preventing or treating an inflammatory disease.

In the present specification, the term "increase of the expression level" means that the expression level or intrinsic function of the Zkscan8 gene is significantly increased by the test substance to such a level that recovery from inflammatory response is detectable as the expression level of the inflammatory response-related factors COX-2, mPGES-1, TNF-α, MMP-1 and MMP-3 is decreased or the activity of the anti-inflammatory factor IL-10 is increased. Specifically, it may mean a state where the expression level is increased by 5% or more, 10% or more, more specifically 20% or more, as compared to a normal control group.

### [Advantageous Effects]

The features and advantages of the present disclosure may be summarized as follows:
(a) The present disclosure provides a composition for preventing or treating an inflammatory disease and a method for screening the same.
(b) Since the Zkscan8 of the present disclosure activates IL-10 by reducing or inhibiting the expression of inflammatory response-related genes (COX-2, mPGES-1, TNF-α, MMP-1, MMP-3), it can be usefully used as a composition for alleviating, preventing or treating an inflammatory disease.

### [Brief Description of Drawings]

FIG. 1a shows a model for the structure of the ZSCAN family.
FIG. 1b shows the cleavage map of pscAAV-Zkscan8.
FIG. 1c schematically shows the structure of a pscAAV-GFP vector and a pscAAV-Zkscan8 vector.
FIG. 2 summarizes the distribution of datasets during embryogenesis (stages E9.5-E15.5).
FIG. 3a shows a result of analyzing the expression pattern of tenogenesis markers and Zkscan8 depending on the stages of embryogenesis.
FIG. 3b shows a result of measuring the expression pattern of tenogenesis markers and Zkscan8 in a normal human tendon (Normal) and the tendon of a patient with rotator cuff disease (Tendinopathy).
FIG. 4a shows the microscopic images of Zkscan8-introduced umbilical cord-derived mesenchymal stem cells (MSC-Zk8) and a control group (MSC-GFP).
FIG. 4b shows a result of quantifying the proliferation ability of Zkscan8-introduced umbilical cord-derived mesenchymal stem cells (MSC-Zk8) and a control group (MSC-GFP).
FIGS. 4c and 4d show a result of measuring the expression of the Zkscan8 gene in Zkscan8-introduced umbilical cord-derived mesenchymal stem cells (MSC-Zk8) and a control group (MSC-GFP) by RT-PCR.
FIG. 4e shows a result of analyzing the expression level of the Zkscan8 protein in Zkscan8-introduced umbilical cord-derived mesenchymal stem cells (MSC-Zk8) and a control group (MSC-GFP) by western blotting.
FIG. 5 shows a result of measuring the expression pattern of inflammation-related genes (IL-1β, 1L-6, TNF-α and MMP-1) for A, B, C and D groups (coculture for 48 hours) by qRT-PCR. All quantitative data are presented as mean ± standard error (S.E.M) (*p < 0.05).
FIG. 6 shows a result of measuring the expression pattern of inflammation-related genes (COX-2, mPGES-1, MMP-3, IL-10) for A, B, C and D groups (coculture for 48 hours) by qRT-PCR. All quantitative data are presented as mean ± standard error (S.E.M) (*p < 0.05).
FIG. 7a shows a result of analyzing the expression level of matrix metalloproteinases (MMPs) MMP-1 and MMP-3 for A, B, C and D groups (coculture for 48 hours) by western blotting.
FIG. 7b shows a result of quantifying the expression level of matrix metalloproteinases (MMPs) MMP-1 and MMP-3 for A, B, C and D groups (coculture for 48 hours) by western blotting. All quantitative data are presented as mean ± standard error (S.E.M) (*p < 0.05).
FIG. 8a shows a result of quantifying the expression level of bFGF for A, B, C and D groups (coculture for 48 hours) by qRT-PCR. All quantitative data are presented as mean ± standard error (S.E.M) (*p < 0.05).
FIG. 8b-FIG. 8d show a result of analyzing the protein secretion level of bFGF, TGF-β2 and HGF for A, B, C and D groups by enzyme-linked immunosorbent assay. All quantitative data are presented as mean ± standard error (S.E.M) (*p < 0.05).
FIG. 9a shows a result of quantifying the expression level of Scx for A, B, C and D groups (coculture for 48 hours) by qRT-PCR. All quantitative data are presented as mean ± standard error (S.E.M) (*p < 0.05).
FIG. 9b shows a result of quantifying the expression level of Mkx for A, B, C and D groups by qRT-PCR. All quantitative data are presented as mean ± standard error (S.E.M) (*p < 0.05).
FIG. 9c shows a result of quantifying the expression level of Egr-1 for A, B, C and D groups by qRT-PCR. All quantitative data are presented as mean ± standard error (S.E.M) (*p < 0.05).
FIG. 9d shows a result of quantifying the expression level of Egr-2 for A, B, C and D groups by qRT-PCR. All quantitative data are presented as mean ± standard error (S.E.M) (*p < 0.05).
FIG. 10 shows a result of analyzing the expression pattern of fat, bone, cartilage, tendon, muscle and nerve cell-related markers in Zkscan8-introduced umbilical cord-derived mesenchymal stem cells (MSC-Zk8) and a control group (MSC-GFP) by RT-PCR.
FIG. 11a shows the macroscopic images of the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. The tissues around the defective region were removed for easier observation of the defect state of the tendon.
FIG. 11b shows a result of analyzing total macroscopic score for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).
FIG. 11c shows a result of analyzing tendon thickness for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).
FIG. 11d shows a result of analyzing swelling/redness of tendon for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).
FIG. 11e shows a result of analyzing inflammation for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).
FIG. 12a shows the H&E staining images of the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment (magnification: x400).
FIG. 12b shows a result of analyzing vascular density for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).
FIG. 12c shows a result of analyzing infiltrated inflammatory cells for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

### [Best Mode]

Hereinafter, the present disclosure will be described in more detail through examples. The examples are provided only to describe the present disclosure more specifically and it will be obvious to those having ordinary knowledge in the art that the scope of the present disclosure is not limited by the examples.

### Examples

### Experimental methods

### Screening and selection of genes

In order to search for biomarkers related with inflammatory diseases, particularly musculoskeletal inflammations, whole transcriptome expression profiling microarray datasets GSE30138 and GSE54207 and RNA sequencing dataset GSE65180 associated with mouse limb tenogenesis were downloaded from the Gene Expression Omnibus (GEO) database (http://www.ncbi.nlm.gov/geo/) of the National Center for Biotechnology Information (NCBI) and analyzed for screening of tendon regeneration candidate genes.

For GSE30138 and GSE54207, the GPL1261 platform (Affymetrix mouse genome 430 2.0 array) was used. And, for GSE65180, the GPL13112 platform [Illumina Hiseq 2000 (*Mus musculus*)] was used. Genes showing similar expression patterns depending on embryonic stages as tenogenesis markers (Tgf-β2, Scx, Mkx, Egr1, Tnmd, Thbs4, Col1, Gag, Tnc), myogenesis markers (Pax3, Pax7, Myf5, Des, MyoD, myogenin, MRF4, myostatin, MHC2), osteogenesis marker (Runx2, Osterix, Alp, Ocn, Opn) and chondrogenesis markers (Comp, Sox9, Chad, Acan, Col2a1) were screened primarily.

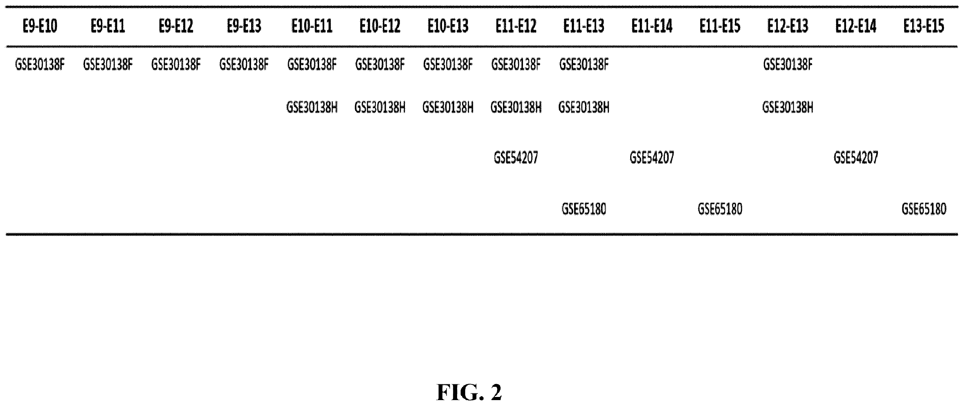

For construction of the protein-protein interaction (PPI) network and investigation of the interaction between genes, analysis was conducted using the TiCoNE and ClueGo plugins of the Cytoscape software (http://www.cytoscape.org/) (FIG. 2). Next, in order to screen hub genes, significant modules closely related in the PPI network such as molecular complex detection (MCODE; degree cutoff = 2.1, node score cutoff = 0.2, k-core = 2, max. depth = 100) and Markov clustering (MCL) were used. Candidate genes were screened by analyzing the relationship of the screened clusters with tendon regeneration through gene ontology (GO) analysis.

### RNA-seq analysis

Total RNAs of a normal tendon (n = 5) and a rotator cuff (n = 6) were extracted using an RNeasy Plus universal kit (Cat. 73404. Qiagen, USA) according to the manufacturer's protocol. The integrity of the total RNAs was identified with an Agilent 2100 bioanalyzer (Agilent Technologies, Santa Clara, CA, USA) and a cDNA library was created using a TruSeq stranded mRNA library prep kit (Cat. 20020594; Illumina, USA) according to the manufacturer's protocol. The pooled library was loaded on HiSeq 2000 at a concentration of 2 nM and then sequenced (v3 SBS chemistry, 76 cycles, paired-end).

### Culturing of human umbilical cord-derived stem cells and tendon cells

All the tissues used in the study were acquired and used under the consent of patients. Umbilical cord and tendon tissues were washed 2-3 times with Ca²⁺- and Mg²⁺-free Dulbecco's phosphate-buffered saline (DPBS, GIBCO, NY, USA) supplemented with antibiotics (100 U/mL penicillin, 100 µg/mL streptomycin sulfate and 0.25 µg/mL amphotericin B (antibiotic-antimycotic solution; Welgene, Daegu, Korea)) in order to remove impurities such as blood, etc.

Umbilical cords were acquired from patients who received Caesarean section. After measuring length and weight, the umbilical cord was cut to about 2-4 mm in length using surgical scissors, and an amount corresponding to 1 g was aligned and inoculated onto a 150 cm² culture dish. After the umbilical cord completely adhered to the culture dish, it was cultured using a culture medium (LG DMEM, 10% fetal bovine serum (FBS; Welgene, Daegu, Korea) and antibiotic-antimycotic solution) at 37 °C while supplying 5% CO₂.

Tendon tissues obtained from a patient with complete rotator cuff tear were cultured at 37 °C for 2 hours with light shaking in HG DMEM (high-glucose Dulbecco's modified Eagle's medium, Welgene, Daegu, Korea) supplemented with 0.3% type 2 collagenase (GIBCO) and antibiotics. Subsequently, after adding a culture medium (HG DMEM, 10% FBS) and antibiotics (antibiotic-antimycotic solution) of the same volume, undissociated tissues were removed using a 100-µm cell filter. Cells recovered by centrifuging (500 g, 15 minutes) at 20 °C were washed twice with a culture medium. The recovered cells were counted by trypan blue exclusion assay. The cells were transferred onto a culture dish at a density of 2×10⁴ to 5×10⁴ cells/cm² and then cultured at 37 °C in a 5% CO₂ incubator.

When the cells grew to fill about 60-80% of the culture dish, they were washed twice with DPBS and adherent cells were detached by treating with trypsin-EDTA (Welgene, Daegu, Korea) containing 0.05% trypsin and 0.53 mM EDTA (ethylenediaminetetraacetic acid) for 3 minutes. Then, the cells were stained and counted by trypan blue exclusion assay. Human umbilical cord-derived stem cells (3,333 cells/cm²) and tendon cells were cultured continuously at a ratio of 1:3.

### Preparation of Zkscan8-introduced umbilical cord-derived mesenchymal stem cells (MSC-Zk8)

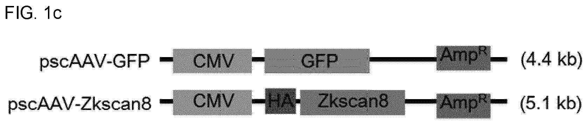

A pscAAV-GFP vector plasmid (Cell Biolabs, CA, USA) was used as a control group vector. After cleaving the GFP region with restriction enzymes BamHI and Sall, Zkscan8 was cloned into the region using primers (FP: 5'-AAGGATCCATGTACCCATACGATGTTCCAGATTACGCTATGGCGGAGGAAAGTC GG-3', RP: 5'-AAGTCGACCTAGACTGAGATAGACTC-3') and BamHI and Sall restriction enzymes. The cleavage map of the pscAAV-Zkscan8 vector is shown in FIG. 1b, and the structures of the prepared pscAAV-GFP and pscAAV-Zkscan8 vectors are schematically shown in FIG. 1c. The sequence of pscAAV-Zkscan8 was analyzed by sequencing. For production of viral packaging stocks, three vectors (target expression vectors, pAAV-RC, pHelper) were introduced into 293 cells according to the manufacturer's instructions. 72 hours later, the culture medium including the cells were collected, frozen and then thawed. An adenovirus including the Zkscan8 gene was obtained by repeating this procedure. The prepared virus was used as a Zkscan8 gene delivery system for human umbilical cord-derived stem cells.

### Analysis of proliferation ability

Proliferation ability was analyzed by measuring the amount of living cells by WST assay. After inoculating the gene-introduced umbilical cord-derived mesenchymal stem cells onto a culture dish at a density of 5×10⁴ cells/cm², 10 µL of Ez-Cytox (DoGEN, Guro, Korea) was added to the culture medium on days 3, 5, 9, 12 and 15. After incubation at 37 °C in a 5% CO₂ incubator for 1 hour, absorbance was measured at a wavelength of 450 nm.

### Coculture of gene-introduced stem cells (MSC-Zk8, MSC-GFP) and tendon cells

In order to evaluate the paracrine ability of Zkscan8-introduced umbilical cord-derived mesenchymal stem cells (MSC-Zk8), they were cocultured with tendon cells. 1.6×10⁴ Zkscan8-introduced umbilical cord-derived mesenchymal stem cells (MSC-Zk8) or control group cells (MSC-GFP) were inoculated onto a culture dish, and 1.1×10⁵ tendon cells were inoculated using an insert (24 wells, 0.4 µm; BD Biosciences, CA, USA). The tendon cells were cultured in a 37 °C incubator for 6 hours under a normal environment of a DMEM-HG culture medium supplemented with antibiotics and 2% FBS or an inflammatory environment of a DMEM-HG culture medium supplemented with antibiotics, 2% FBS and 10 ng/mL interleukin-1 beta (IL-1β; Peprotech, NJ, USA).

6 hours later, a total of four coculture samples were prepared by transferring the insert holding the tendon cells cultured under the normal environment or inflammatory environment to the culture dish holding the Zkscan8-introduced umbilical cord-derived mesenchymal stem cells (MSC-Zk8) or the control group (MSC-GFP).

A (IL-1β, MSC; -. -): insert holding the tendon cells cultured under the normal environment, B (IL-1β, MSC; +. -): insert holding the tendon cells cultured under the inflammatory environment, C (IL-1β, MSC-GFP; +. +): insert holding the tendon cells cultured under the inflammatory environment and culture dish holding the control group (MSC-GFP), D (IL-1β, MSC-Zk8; +. +): insert holding the tendon cells cultured under the inflammatory environment and culture dish holding the Zkscan8-introduced umbilical cord-derived mesenchymal stem cells (MSC-Zk8) cells. The tendon cells and the gene-introduced stem cells (MSC-Zk8, MSC-GFP) were cocultured as stated above. The coculture was performed for 48 hours using a DMEM-LG culture medium supplemented with antibiotics, 2% FBS and 10 ng/mL interleukin-1 beta (IL-1β; Peprotech, NJ, USA).

### Isolation of RNAs from Zkscan8-introduced umbilical cord-derived mesenchymal stem cells (MSC-Zk8)

In order to investigate the expression of various genes upon introduction of Zkscan8, total RNAs were extracted using a HiYield total RNA mini kit (Real Biotech Corporation, Taiwan). After measuring absorbance at 260 nm and 280 nm using a spectrophotometer (NanoDrop, DE, USA), the total RNAs were quantified. Then, cDNAs were synthesized from 1 µg of the total RNAs using a Superscript II reverse transcriptase (Invitrogen, CA. USA).

### Reverse transcription polymerase chain reaction (RT-PCR)

Maxime^{™} PCR PreMix (i-StarTaq, iNtRON, Sungnam, Korea) was used to conduct reverse transcription polymerase chain reaction (RT-PCR). 16 µL of RNase-free distilled water, 2µL of cDNAs and 1 µL of primers per each were added and the reaction was conducted under the following conditions. Pre-denaturation was performed at 95 °C for 30 minutes, denaturation at 95 °C for 30 seconds, annealing at 55 °C for 30 seconds, and extension at 72 °C for 1 minute. After repeating this procedure for a total of 32 times, followed by cooling at 72 °C for 5 minutes, the bands of the obtained PCR product were observed on 1% agarose gel.

**[Table 1]**

| Target genes | | Primer sequences (5' → 3') | Product length (bp) |
|---|---|---|---|
| Zkscan8 | SEQ ID NO 4 | CGGAGGAAAGTCGGAAACCA | 198 |
| Zkscan8 | SEQ ID NO 5 | CTTCCCGTGGACCAAGAGTC | 198 |
| PPARγ2 | SEQ ID NO 6 | TTGGTGACTTTATGGAGCCC | 311 |
| PPARγ2 | SEQ ID NO 7 | CATGTCTGTCTCCGTCTTCT | 311 |
| aP2 | SEQ ID NO 8 | AAGAAGTAGGAGTGGGCTTT | 285 |
| aP2 | SEQ ID NO 9 | CCACCACCAGTTTATCATCC | 285 |
| Osteopontin | SEQ ID NO 10 | GAGACCCTTCCAAGTAAGTC | 354 |
| Osteopontin | SEQ ID NO 11 | GATGTCCTCGTCTGTAGCAT | 354 |
| ALP | SEQ ID NO 12 | TGGAGCTTCAGAAGCTCAAC | 454 |
| ALP | SEQ ID NO 13 | ATCTCGTTGTCTGAGTACCA | 454 |
| Sox6 | SEQ ID NO 14 | AACATGTGGCCTCCCATCTG | 300 |
| Sox6 | SEQ ID NO 15 | TCAGTGTGTCCACCACATCG | 300 |
| Aggrecan | SEQ ID NO 16 | TCAGGAGGGCTGGAACAAGT | 350 |
| Aggrecan | SEQ ID NO 17 | GGAGGTGGTAATTGCAGGGA | 350 |
| Mkx | SEQ ID NO 18 | GGCCACGAACACTACCATGA | 175 |
| Mkx | SEQ ID NO 19 | AGCTGCGCTTTCACCCTTAT | 175 |
| Egr-1 | SEQ ID NO 20 | CCAGTGGAGTCCTGTGATCG | 206 |
| Egr-1 | SEQ ID NO 21 | TCGCTCCTGGCAAACTTTCT | 206 |
| Egr-2 | SEQ ID NO 22 | TCTTCCTCTCTGGCCTACCC | 400 |
| Egr-2 | SEQ ID NO 23 | GTCTGTTGGGGTACTTGCGA | 400 |
| COL1a1 | SEQ ID NO 24 | AGTGGTTTGGATGGTGCCAA | 170 |
| COL1a1 | SEQ ID NO 25 | GCACCATCATTTCCACGAGC | 170 |
| COL3a1 | SEQ ID NO 26 | CTTCTCTCCAGCCGAGCTT | 191 |
| COL3a1 | SEQ ID NO 27 | CCAGTGTGTTTCGTGCAACC | 191 |
| Desmin | SEQ ID NO 28 | GAGGAAATCCGGCACCTCAA | 253 |
| Desmin | SEQ ID NO 29 | CATCCCCGTGTCTCGATGGTC | 253 |
| NEFL | SEQ ID NO 30 | CTGGAAATCGAAGCATGCCG | 363 |
| NEFL | SEQ ID NO 31 | GCGGGTGGACATCAGATAGG | 363 |
| GAPDH | SEQ ID NO 32 | AAATCCCATCACCATCTTCCA G | 313 |
| GAPDH | SEQ ID NO 33 | CATGAGTCCTTCCACGATACC | 313 |

### Quantitative reverse transcription polymerase chain reaction (quantitative RT-PCR)

The expression of the following genes was quantified in real time by quantitative reverse transcription polymerase chain reaction (quantitative RT-PCR; qRT-PCR) using Go Taq^{™} probe qPCR and RT-qPCR systems (Promega, WI, USA), TaqMan^{™} Gene Expression Assays (Applied Biosystems, Foster City, CA, USA) and LightCycler 480 (Roche Applied Science, Mannhein, Germany); Scx (Hs03054634_g1), Mkx (Hs00543190_m1), Egr-1 (Hs00152928_m1), Egr-2 (Hs00166165_m1), Thbs4 (Hs00170261_m1), GAPDH (Hs99999905_m1). The polymerase chain reaction was conducted as follows. After repeating 50 cycles of pre-denaturation at 95 °C for 10 minutes, denaturation at 95 °C for 15 seconds, annealing at 60 °C for 1 minute and extension at 72 °C for 4 seconds, the reaction mixture was cooled at 40 °C for 30 seconds. Melting curve analysis was performed using the 2-ΔCt calculation method, and the result of qRT-PCR was analyzed with reference to the expression of GAPDH [Livak KJ, Schmittgen TD. Analysis of relative gene expression data using real-time quantitative PCR and the 2-ΔΔCt method. Methods. 2001, 25:402-408].

### Isolation of proteins and western blotting

A tendon cell lysate was obtained from tendon cells as follows. After adding a lysis buffer (PRO-PREPTM, iNtRON, Sungnam, Korea) to tendon cell pellets, they were disrupted by vigorously vortexing for 10 seconds for 10 or more times. The resulting solution was centrifuged at 4 °C and at 13,000 rpm for 20 minutes, and a tendon cell lysate was obtained by recovering the supernatant. The tendon cell lysate was subjected to BCA for quantification of protein contents. The tendon cell lysates (10 µg) of the same concentration were loaded onto 10% acrylamide gel and proteins were separated based on size by electrophoresis (SDS-PAGE). The separated proteins were sufficiently soaked with a 1x transfer buffer (20% methanol, 0.025 M Tris base and 0.19 M glycine) and then transferred to a PVDF membrane by flowing current at 100 V for 90 minutes. The PVDF membrane was blocked for 1 hour in a 1x TBS-T solution (10 mM Tris pH7.5, 100 mM NaCl, 0.1% Tween 20) supplemented with 5% skim milk. Then, after adding primary antibodies to the 1x TBS-T solution at a ratio of 1:1000, reaction was performed by stirring at 4 °C. The used primary antibodies are as follows: Zkscan8 antibody (STJ26239, St John's Laboratory), HA antibody (ab9110, Abcam), MMP-1 antibody (MAB901, R&D Systems), MMP-3 antibody (MAB905, R&D Systems), vinculin antibody (13901, Cell Signaling Technology) and β-actin antibody (ab170325, Abcam). The PVDF membrane reacted with the primary antibodies were washed sufficiently with 1x TBS-T for 10 minutes for 3 times or more. Then, after adding secondary antibodies (diluted to 1:20,000) to the 1x TBS-T solution, reaction was performed for 1 hour by stirring at room temperature. The used secondary antibodies are as follows: goat anti-mouse IgG-HRP (SA001-500, GenDEPOT) and goat anti-rabbit IgG-HRP (7074S, Cell Signaling). After washing 3 times with a 1x TBS-T solution for 10 minutes and treating with ECL, the PVDF membrane was imaged with ImageQuant LAS4000 mini (GE Healthcare Life Sciences, Little Chalfont, UK).

### Enzyme-linked immunosorbent assay (ELISA)

After the coculture, the culture was centrifuged at 4 °C and at 13,000 rpm for 20 minutes and the supernatant was used for experiment. Enzyme-linked immunosorbent assay was performed using bFGF (RayBio human bFGF ELISA kit, GA, USA), HGF (DHG00; R&D system, INC., Minneapolis, MN) and TGF-β2 (DB250; R&D system, INC., Minneapolis, MN) according to the manufacturer's protocol.

### Design of animal experiment

All animal care and experimental procedures were approved by and conducted in accordance with the institutional experimental animal research committee (IACUC_2019_0044). 32 male Sprague-Dawley rats (12-week-old, 340-360 g) were divided into four groups (1) normal group (Normal); 2) physiological saline group (Saline); 3) umbilical cord-derived mesenchymal stem cell group (US-MSC); 4) Zkscan8-introduced umbilical cord-derived mesenchymal stem cell group (Zkscan8+)).

The rats were anesthetized using Zoletil (30 mg/kg) and Rompun (10 mg/kg), and only the left shoulders of the rats were used in all the experiments. Surgery was conducted after slightly pressing the rat's sole with a fingernail to check if the anesthesia was successful. Then, the skin in front of the acromion of the left shoulder was incised by 2 cm. After exposing the supraspinatus tendon by incising the trapezius muscle and deltoid muscle attached to the acromion, a round full-thickness rupture tendon injury was made at a distance of 1 mm from the cartilage region of the supraspinatus tendon and the humeral head using a biopsy punch (BP-20F, Kai Medical Europe GmbH, Bremen, Germany) with a diameter of 2 mm (about 50% or larger of the tendon width). Then, 2) 10 µL of physiological saline, 3) umbilical cord-derived mesenchymal stem cells (1×10⁶ cells/10 µL physiological saline) or 4) Zkscan8-introduced umbilical cord-derived mesenchymal stem cells (1×10⁶ cells/10 µL physiological saline) was injected to both sides of the ruptured tendon in two divided dosages using a 30-G needle. After suturing the trapezius and deltoid muscles with a 4-0 Vicryl suture (W9074, Ethicon, Cincinnati, OH, USA) and suturing the skin with Black Silk (SK439, AILee, Busan, Korea), the wound site was disinfected. After the surgery, the rats were allowed free cage activity.

The rats were sacrificed at weeks 2 and 4 after the surgery. The supraspinatus tendon of the rats was harvested and used for macroscopic, histological and biomechanical evaluation.

### Macroscopic evaluation

At 2 and 4 weeks after the surgery, the rats were sacrificed in a carbon dioxide chamber. The supraspinatus tendon of the rats was harvested along with the humeral head without removing the supraspinatus muscle. For macroscopic evaluation of tendon regeneration, Stoll's modified semi-quantitative evaluation system was used [Stoll C, John T, Conrad C et al. Healing parameters in a rabbit partial tendon defect following tenocyte/biomaterial implantation. Biomaterials 2011; 32(21): 4806-4815]. The 12 parameters in the system were tendon rupture (breakage at the defective site), inflammation (swelling/redness or inflammation), tendon surface (tendon surface is not smooth but rough and uneven), neighboring tendon (the color, thickness and outer surface of the of tendon near the defective site turn abnormal), level of the defect (the defective site is filled and bulges as compared to the neighboring tendon), defect size (defect size is increased to 3 mm or larger), swelling/redness of tendon (the injured tendon turns red or swells), connection surrounding tissue and slidability (the injured tendon congeals with neighboring tissue without sliding (being separated) smoothly), tendon thickness (tendon thickness is increased as compared to the original thickness), color of tendon (bright white tendon has turn opaque and dark red), single strain of muscle (the supraspinatus tendon is intermixed with neighboring muscle and tissue, rather than only with the supraspinatus muscle), and transition of the construct to the surrounding healthy tissue (the connection between the defective site and neighboring healthy tendon is not smooth; the beginning of defect is clearly distinguished). Each parameter was given 0 or 1 point except for tendon swelling/redness (0 to 2) and tendon thickness (0 to 3). Therefore, the total macroscopic score varied between 0 (normal tendon) and 15 (most severe injury).

### Histological evaluation

After the macroscopic evaluation, the harvested tissues were immediately fixed in 4% (w/v) paraformaldehyde (PFA; Merck, Germany) for 24 hours, followed by decalcification in 10% EDTA (ethylendiaminetetraacetic acid, Sigma-Aldrich, St Louis, MO, USA) for 2 days. After the decalcification, the tissues were dehydrated through an increasing series of ethanol gradient and defatted in chloroform. The fixed tissues were embedded in paraffin blocks and carefully trimmed to the appropriate middle site of tendon using a microtome, cut into 4-cm-thick sections and attached onto slides.

A randomly selected slide was stained with hematoxylin and eosin (H&E) and analyzed with an optical microscope (U-TVO 63XC; Olympus Corp., Japan). H&E-stained slides were used to evaluate vessel density (for evaluation of increased blood vessel formation due to tendon tissue damage) according to Fernandez-Sarmiento's method [Fernandez-Sarmiento JA, Dominguez JM, Granados MM et al. Histological study of the influence of plasma rich in growth factors (PRGF) on the healing of divided Achilles tendons in sheep. J Bone Joint Surg Am 2013; 95 (3): 246-255]. The vessel density was evaluated as the number of blood vessels per area and was analyzed using the ImageJ software with installed NII plugin (National Institutes of Health, MD, USA). Five regions were analyzed for each slide and the average was used finally.

For evaluation of infiltrated inflammatory cells in tissues, the degree of infiltration of the inflammatory cells was evaluated using a 0-4 grading scale: 0 (no infiltration), 1 (slight infiltration), 2 (moderate infiltration), 3 (strong infiltration) and 4 (severe infiltration) [Chen L, Liu JP, Tang KL et al. Tendon derived stem cells promote platelet-rich plasma healing in collagenase-induced rat Achilles tendinopathy. Cell Physiol Biochem 2014; 34 (6): 2153-2168.]. All tissues were evaluated twice.

### Statistical analysis

All data were shown as mean ± SD. The data were analyzed by one-way analysis of variance (ANOVA) and post-hoc analysis was performed using Bonferroni multiple comparison test. T-test was used to compare two groups. All statistical analyses were performed using the SPSS software version 23 (IBM). Differences of p < 0.05 were considered statistically significant.

### Experimental results

### Analysis of gene expression pattern and PPI network during embryogenesis

For analysis of the expression patterns of tenogenesis, myogenesis, chondrogenesis and osteogenesis markers during embryonic stages, the expression patterns of genes in embryonic stages E9.5-E15.5 were analyzed from whole transcriptome expression profiling datasets GSE30138, GSE54207 and GSE65180. FIG. 2 summarizes the distribution of the datasets during embryogenesis (stages E9.5-E15.5).

Because analysis was conducted using three datasets, the similarity of expression patterns to marker genes was compared based on distance scores of the genes. 0 point was given if the expression pattern was identical to that of the marker genes in the three datasets. 0.33 point and 0.67 were given, respectively, if it is different in one and two datasets when the expression pattern began to increase. And, -0.33 point and -0.67 were given, respectively, if it is different in one and two datasets when the expression pattern began to decrease. Among the 9,163 DEGs, 149 genes with total distance scores of ±0.33 or lower, i.e., those showing difference in expression patterns in only one embryogenic stage, were screened primarily. The primarily screened candidate genes were subjected to PPI network, functional enrichment and module analyses while adjusting degree cut-off.

As a result of the protein-protein interaction (PPI) network analysis, three sub-clusters were identified. 99 genes were screened secondarily by excluding well-known genes. Then, 42 genes functionally related to tendon regeneration were screened tertiarily through gene ontology (GO). The tertiarily screened 42 genes were used for the final screening of genes related with tendon regeneration.

### Comparison of expression pattern of tenogenesis markers and Zkscan8 depending on embryogenic stages

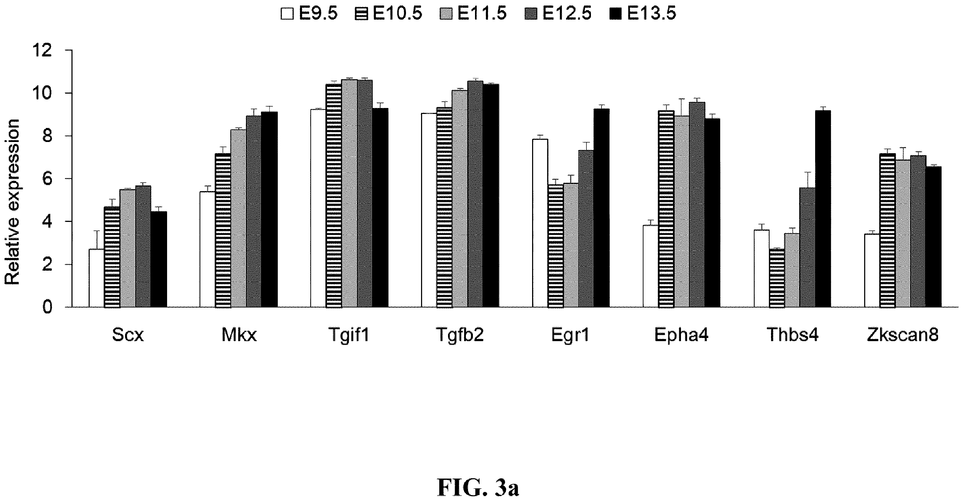

FIG. 3a shows a result of analyzing the expression pattern of tenogenesis markers and Zkscan8 depending on the stages of embryogenesis.

The expression pattern of the Zkscan8 gene was compared with that of tenogenesis markers depending on embryogenic stages to analyze their similarity. As shown in FIG. 3a, Scx and Tgif1 were increased continuously from E9.5 and were decreased at E13.5. Mkx was increased continuously until E13.5, and Tgf-β2 showed consistently high expression levels from E9.5 to until E13.5. Egr1 and Thbs4 were decreased at E10.5-E11.5 as compared to at E9.5 and then were increased again from E12.5. Epha4 was increased remarkably from E10.5. The expression of Zkscan8 was increased at E10.5 and the expression pattern was generally similar to that of the tendon markers.

### Analysis of Zkscan8 expression pattern in normal tendon and tendon with rotator cuff disease

The expression patterns of tenogenesis markers and the Zkscan8 gene in the normal tendon obtained from a normal human and the tendon obtained from a patient with rotator cuff disease were investigated.

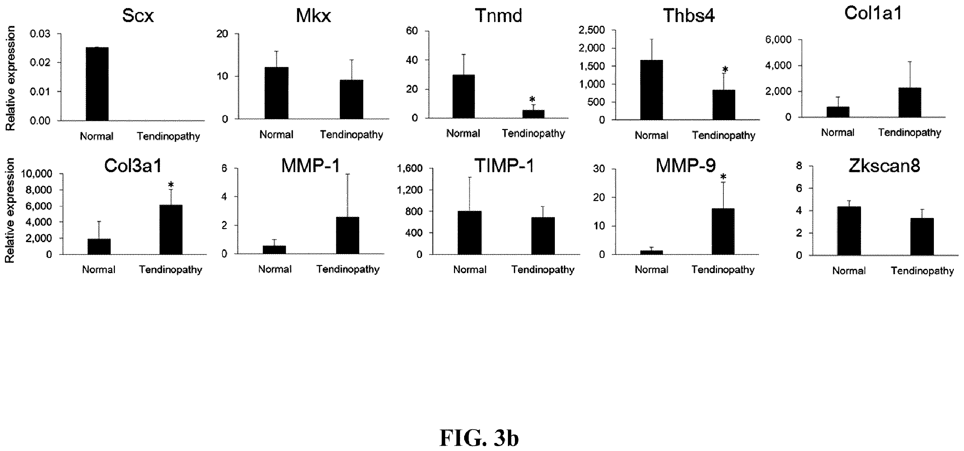

FIG. 3b shows a result of measuring the expression pattern of the tenogenesis markers and Zkscan8 in the normal human tendon (Normal) and the tendon of a patient with rotator cuff disease (Tendinopathy).

As shown in FIG. 3b, the expression of Tnmd and Thbs4, known as tenogenesis markers, was increased significantly in the tendon with rotator cuff disease by about 5.4 times and 2 times, respectively. The expression of Col3a1 was increased significantly in the tendon with rotator cuff disease by 3.2 times as compared to the normal tendon. In addition, the expression of MMP-9 was also increased by 11.8 times in the tendon with rotator cuff disease as compared to the normal tendon. However, the expression of the Zkscan8 gene was specifically in the tendon with rotator cuff disease as compared to the normal tendon. Through this, it was confirmed that Zkscan8 can be used as a gene for preventing or treating rotator cuff disease. Zkscan8 was selected finally.

### Characterization of Zkscan8-introduced umbilical cord-derived mesenchymal stem cells (MSC-Zk8)

A pscAAV-GFP vector, which is a control group vector, and a pscAAV vector wherein Zkscan8 is inserted, in are schematically shown in FIG. 1c. Umbilical cord-derived mesenchymal stem cells into which pscAAV-GFP and pscAAV-Zkscan8 were introduced were prepared as described above, and the characteristics of the Zkscan8-introduced umbilical cord-derived mesenchymal stem cells (MSC-Zk8) were compared with a control group (MSC-GFP). For this, cell morphology and proliferation ability were compared.

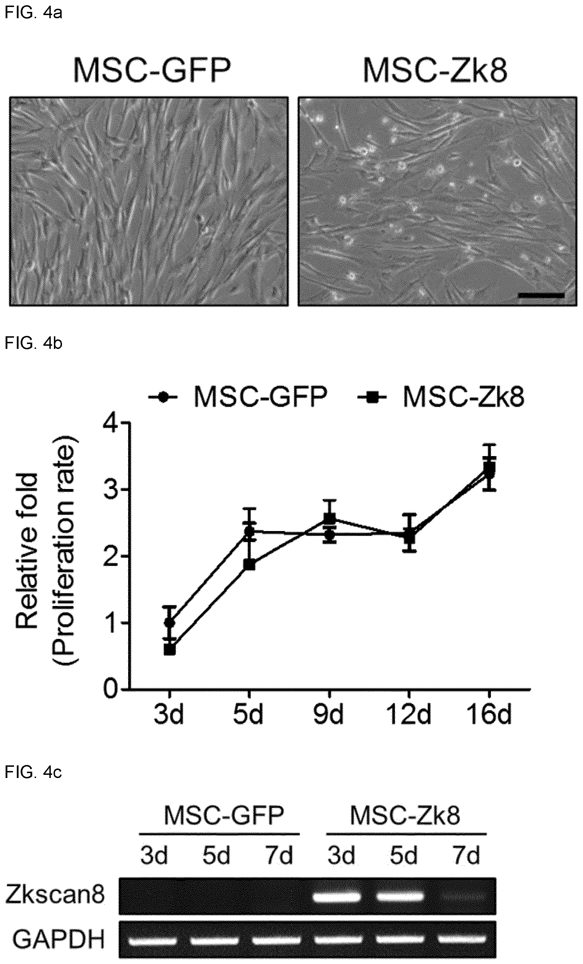

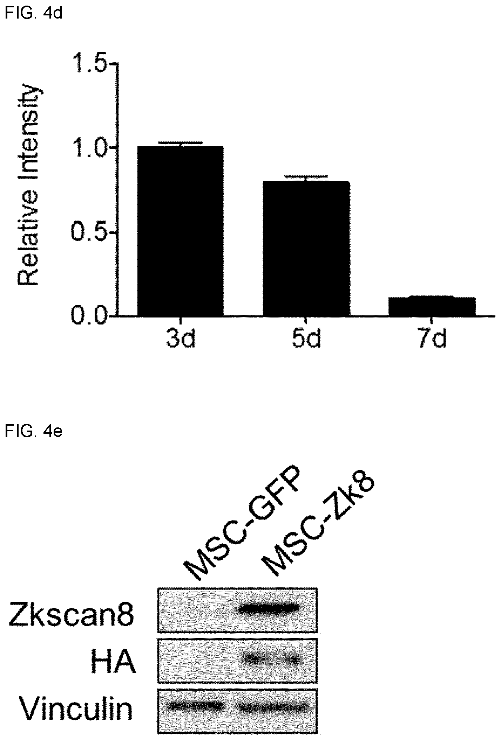

FIG. 4 shows the result of analyzing the characteristics (cell morphology and proliferation ability) of the Zkscan8-introduced umbilical cord-derived mesenchymal stem cells (MSC-Zk8) and the control group (MSC-GFP). FIG. 4a shows the microscopic images of the Zkscan8-introduced umbilical cord-derived mesenchymal stem cells (MSC-Zk8) and the control group (MSC-GFP), FIG. 4b shows a result of quantifying the proliferation ability of the Zkscan8-introduced umbilical cord-derived mesenchymal stem cells (MSC-Zk8) and the control group (MSC-GFP), FIGS. 4c and 4d show a result of measuring the expression of the Zkscan8 gene in the Zkscan8-introduced umbilical cord-derived mesenchymal stem cells (MSC-Zk8) and the control group (MSC-GFP) by RT-PCR, and FIG. 4e shows a result of analyzing the expression of the Zkscan8 protein in the Zkscan8-introduced umbilical cord-derived mesenchymal stem cells (MSC-Zk8) and the control group (MSC-GFP) by western blotting.

As shown in FIGS. 4a and 4b, the Zkscan8-introduced umbilical cord-derived mesenchymal stem cells (MSC-Zk8) and the control group (MSC-GFP) showed the characteristic morphology of fibroblasts. That is to say, the two cells were found to have no difference in proliferation ability.

As shown in FIGS. 4c-4e, the expression level of Zkscan8 over time was analyzed by extracting total RNAs and proteins from the Zkscan8-introduced umbilical cord-derived mesenchymal stem cells (MSC-Zk8) and the control group (MSC-GFP). The relative expression pattern was shown as compared to the control group (MSC-GFP).

From the RT-PCR result, it can be seen that Zkscan8 was overexpressed in the Zkscan8-introduced umbilical cord-derived mesenchymal stem cells (MSC-Zk8) and the control group (MSC-GFP) from 2 days after the introduction of the gene and the expression was decreased after 3 days RT-PCR. After 5 and 7 days, the expression was decreased significantly by about 20.6 ± 4.3% and 89.70 ± 1.3%, respectively.

From the western blotting result, it was confirmed using Zkscan8 and HA antibodies that the Zkscan8 protein was overexpressed in the Zkscan8-introduced umbilical cord-derived mesenchymal stem cells (MSC-Zk8) on day 2 after the introduction of Zkscan8. Accordingly, it was confirmed that human umbilical cord-derived mesenchymal stem cells overexpressing the Zkscan8 protein were prepared successfully.

### Evaluation of effect of paracrine secretion of Zkscan8-introduced umbilical cord-derived mesenchymal stem cells (MSC-Zk8) on inflammation-related genes

Adult stem cells can reduce inflammation at the disease site and promote tissue regeneration by secreting various cytokines related with healing. The paracrine ability of the Zkscan8-introduced umbilical cord-derived mesenchymal stem cells (MSC-Zk8) was evaluated by inducing an inflammatory environment with cytokine IL-1β, coculturing the Zkscan8-introduced umbilical cord-derived mesenchymal stem cells (MSC-Zk8) with tendon cells and then investigating the change in the expression of inflammation-related factors in the tendon cells.

After extracting total RNAs from the tendon cells of A, B, C and D groups (coculture for 48 hours), the expression of inflammation-mediating factors (interleukin 1 beta; IL-1β, interleukin 6; IL-6, cyclooxygenase; COX-2, microsomal prostaglandin E synthase-1; mPGES-1, tumor necrosis factor-alpha; TNF-α) and an inflammation-inhibiting factor (interleukin-10; IL-10) was analyzed by qRT-PCR.

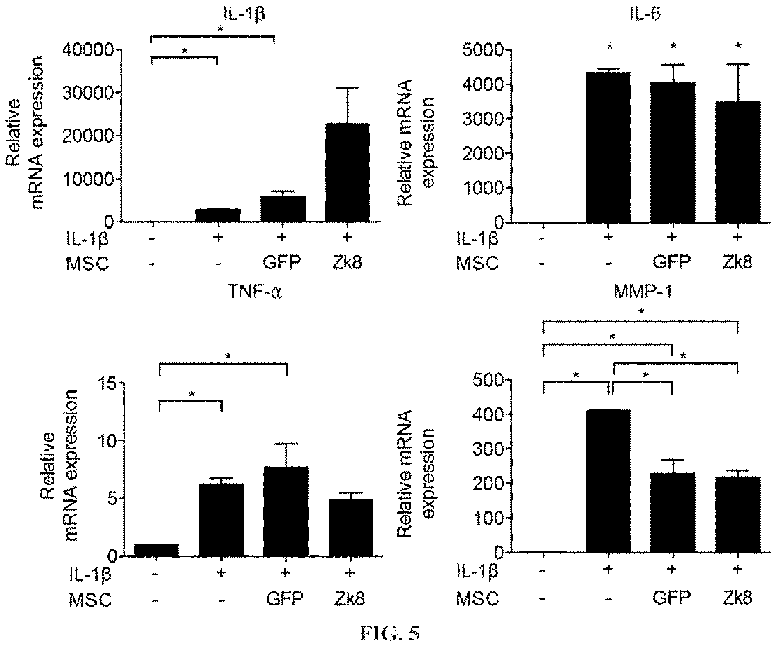

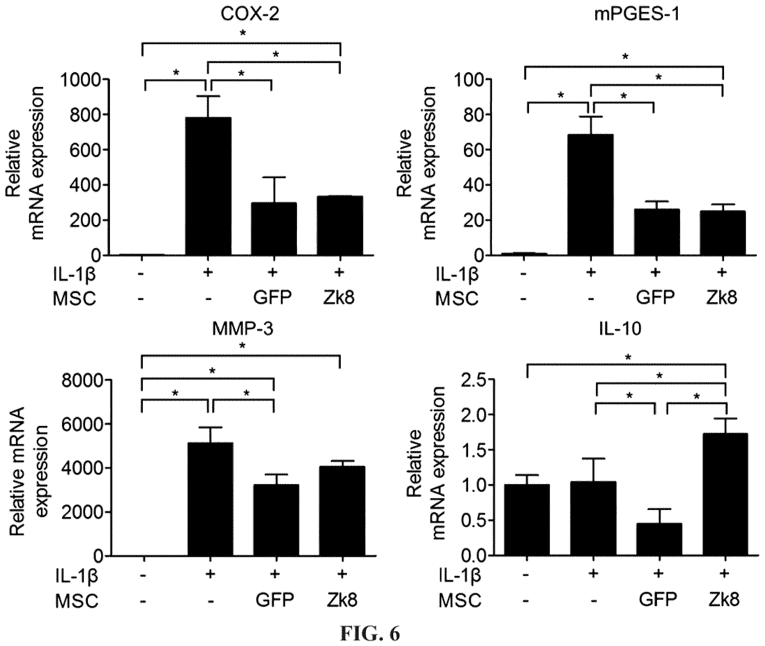

FIG. 5 shows a result of measuring the expression pattern of inflammation-related genes (IL-1β, 1L-6, TNF-α and MMP-1) for A, B, C and D groups (coculture for 48 hours), and FIG. 6 shows a result of measuring the expression pattern of inflammation-related genes (COX-2, mPGES-1, MMP-3, IL-10) for A, B, C and D groups (coculture for 48 hours).

The relative expression of other groups was calculated with reference to the gene expression in the tendon cells under the normal environment (A). As shown in FIG. 5 and FIG. 6, the expression of the inflammation-mediating factors such as IL-1β, IL-6, COX-2, mPGES-1 and TNF-α was increased significantly by 2886.57 ± 56.59, 4330.20 ± 106.11, 781.44 ± 122.87, 68.48 ± 10.43 and 6.20 ± 0.52 times, respectively, in the tendon cells exposed to the inflammatory environment (B). Through this, it can be seen that inflammation was induced in the tendon cells exposed to the inflammatory environment.

In contrast, the expression of COX-2, mPGES-1 and TNF-α was decreased by 57%, 64% and 21%, respectively, when the Zkscan8-introduced umbilical cord-derived mesenchymal stem cells (MSC-Zk8) were cocultured with the tendon cells (D). In addition, when the Zkscan8-introduced umbilical cord-derived mesenchymal stem cells (MSC-Zk8) were cocultured with the tendon cells (D), the expression of the inflammation-inhibiting factor IL-10 was significantly increased as compared to the B group. The inflammation-inhibiting factor IL-10 can help tissue regeneration by acting as an antagonist to TNF-α. It was confirmed that the Zkscan8 of the present disclosure can increase the production of IL-10 via the Cox-2-PGE2 pathway, promote bone formation by adult stem cells, and stabilize the microenvironment for healing by inhibiting the proliferation of immune cells.

Matrix metalloproteinases (MMPs) affect tissue remodeling during the initial phase of healing by degrading damaged tissues. However, because continued activity can degrade the extracellular matrix, misregulation of MMPs can cause the inflammation of tendon on the contrary. Therefore, the expression pattern of the mRNAs and proteins of MMP-1 and MMP-3 when the Zkscan8-introduced umbilical cord-derived mesenchymal stem cells (MSC-Zk8) were cocultured with tendon cells (D) was investigated by western blotting.

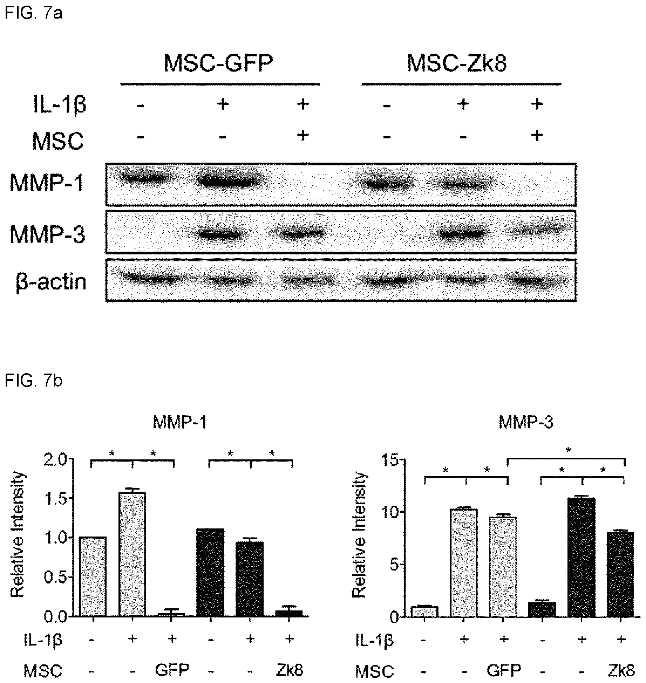

FIG. 7a shows a result of analyzing the expression level of matrix metalloproteinases (MMPs) MMP-1 and MMP-3 for A, B, C and D groups (coculture for 48 hours) by western blotting, and FIG. 7b shows a result of quantifying the expression level of matrix metalloproteinases (MMPs) MMP-1 and MMP-3 for A, B, C and D groups (coculture for 48 hours) by western blotting. All quantitative data are presented as mean ± standard error (S.E.M) (*p < 0.05).

As shown in FIG. 7a and FIG. 7b, the expression of the proteins of MMP-1 and MMP-3 was increased when inflammation was induced in the tendon cells (B). However, the expression of MMP-1 and MMP-3 was decreased significantly when the Zkscan8-introduced umbilical cord-derived mesenchymal stem cells (MSC-Zk8) were coculture with the tendon cells (D). Particularly, it was confirmed that expression of the MMP-3 protein decreased significantly when the Zkscan8-introduced umbilical cord-derived mesenchymal stem cells (MSC-Zk8) were cocultured (D) as compared to when the control group (MSC-GFP) was cocultured.

### Evaluation of effect of paracrine secretion of Zkscan8-introduced umbilical cord-derived mesenchymal stem cells (MSC-Zk8) on growth factor-related genes

bFGF plays an important role in angiogenesis. It is a growth factor necessary for supplying blood to treat tendon. The expression of bFGF is increased during the early stage of healing. Therefore, the expression pattern of growth factors (bFGF, TGF-β2 and HGF) in tendon cells treated with the Zkscan8-introduced umbilical cord-derived mesenchymal stem cells (MSC-Zk8) was investigated.

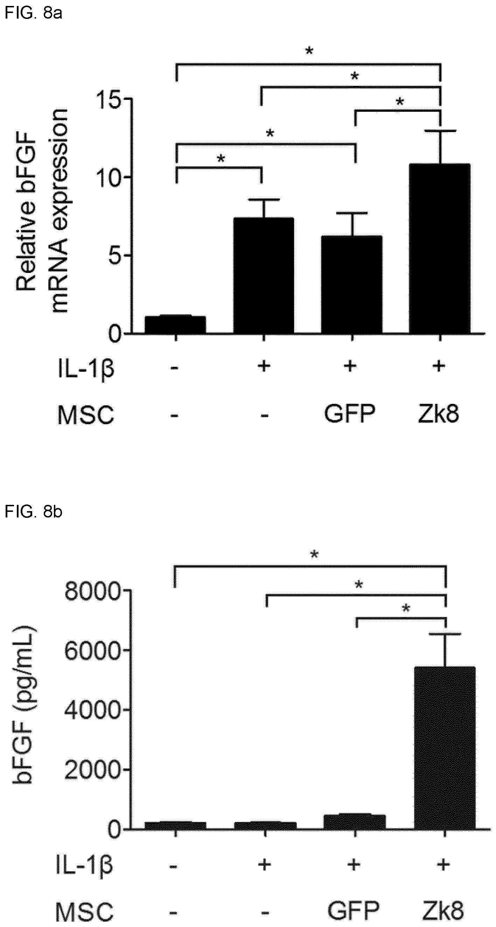

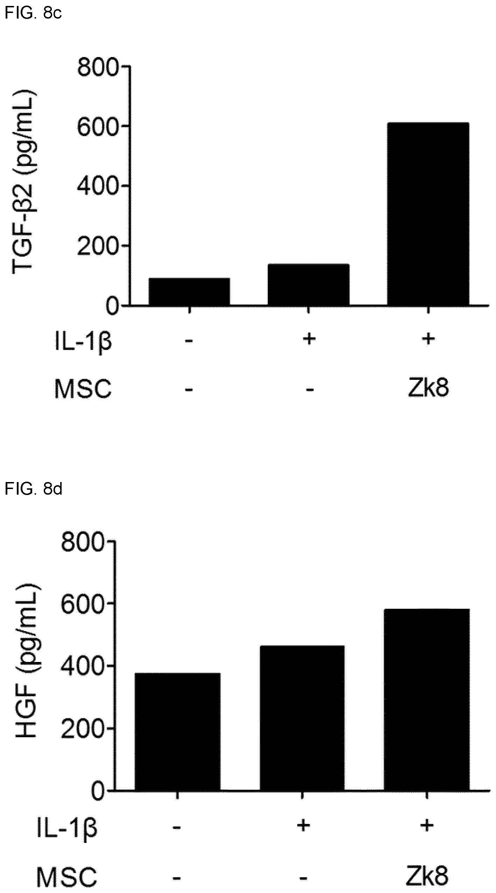

FIG. 8a shows a result of quantifying the expression level of bFGF for A, B, C and D groups (coculture for 48 hours) by qRT-PCR. All quantitative data are presented as mean ± standard error (S.E.M) (*p < 0.05).

As shown in FIG. 8a, the bFGF expression was increased by 7.35 ± 1.22 times when inflammation was induced in the tendon cells (B), and was increased by 10.80 ± 2.20 times when the Zkscan8-introduced umbilical cord-derived mesenchymal stem cells (MSC-Zk8) were cocultured with the tendon cells (D).

The expression level of bFGF, TGF-β2 and HGF in the A, B, C and D groups was investigated by ELISA. A commercially available ELISA kit (Quantikine^{™}, Research & Diagnostic Systems, Minneapolis, MN) was used. Briefly, a sample was put in a well coated with bFGF, TGF-β2 or HGF monoclonal antibody and then stored at room temperature for 2 hours. After washing unbound proteins and adding a substrate solution, absorbance was measured at 450 nm. Measurement for each sample was made multiple times and then averaged.

FIGS. 8b-8d show a result of analyzing the protein secretion level of bFGF, TGF-β2 and HGF for A, B, C and D groups by enzyme-linked immunosorbent assay. All quantitative data are presented as mean ± standard error (S.E.M) (*p < 0.05).

As shown in FIGS. 8b-8d, there was no difference in the bFGF expression level of the tendon cells under the normal environment (A) and the inflammation-induced tendon cells (B). The expression level of bFGF in the tendon cells treated with the control group (MSC-GFP) (C) was 443.56 ± 66.38 pg/mL, and the bFGF expression level in the tendon cells treated with the Zkscan8-introduced umbilical cord-derived mesenchymal stem cells (MSC-Zk8) (D) was 5409.56 ± 1146.73 pg/mL. This means that the bFGF expression level was increased by 26.55 times as compared to the inflammation-induced tendon cells (B) and by 12.20 times or higher as compared to the tendon cells treated with the control group (MSC-GFP) (C).

In addition, the secretion amount of TGF-β2 was 4.52 times or more and the secretion amount of HGF was 1.26 times or more in the tendon cells treated with Zkscan8-introduced umbilical cord-derived mesenchymal stem cells (MSC-Zk8) (D) as compared to the inflammation-induced tendon cells (B).

### Evaluation of effect paracrine secretion of Zkscan8-introduced umbilical cord-derived mesenchymal stem cells (MSC-Zk8) on tendon-related genes

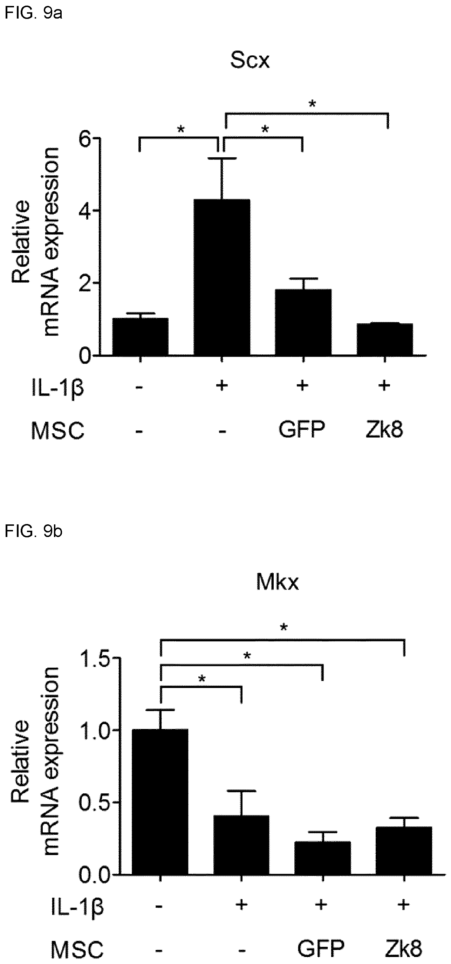

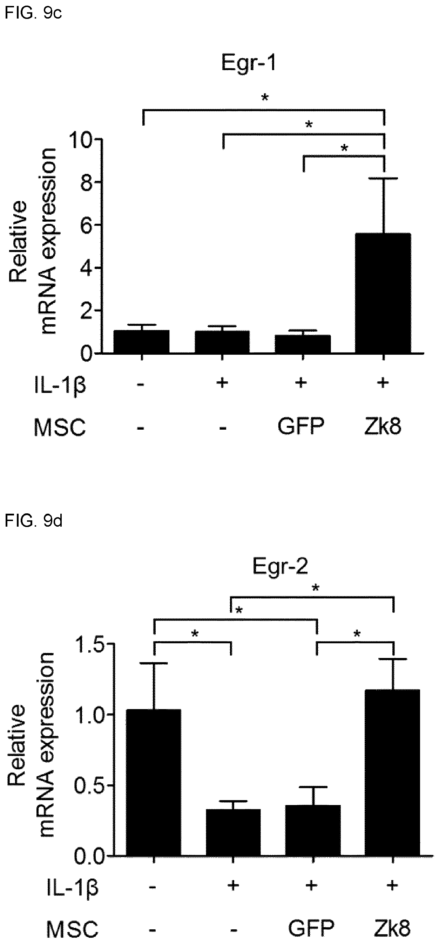

FIG. 9a shows a result of quantifying the expression level of Scx for A, B, C and D groups (coculture for 48 hours) by qRT-PCR, FIG. 9b shows a result of quantifying the expression level of Mkx for A, B, C and D groups by qRT-PCR, FIG. 9c shows a result of quantifying the expression level of Egr-1 for A, B, C and D groups by qRT-PCR, and FIG. 9d shows a result of quantifying the expression level of Egr-2 for A, B, C and D groups by qRT-PCR. All quantitative data are presented as mean ± standard error (S.E.M) (*p < 0.05).

As shown in FIGS. 9a-9d, it was confirmed that Scx expression was significantly increased by 4.30 ± 1.17 times when inflammatory response was induced in the tendon cells (B). In contrast, the Scx expression was significantly decreased when the Zkscan8-introduced umbilical cord-derived mesenchymal stem cells (MSC-Zk8) were cocultured with the tendon cells (D) Scx.

The expression of Mkx was significantly decreased when inflammatory response was induced in the tendon cells (B), and there was little change in the expression level when the Zkscan8-introduced umbilical cord-derived mesenchymal stem cells (MSC-Zk8) were cocultured with the tendon cells (D).

The expression of Egr-1 was not changed when the tendon cells were cocultured with the control group (MSC-GFP) (C), but was increased significantly by 5.57 ± 2.63 times when the Zkscan8-introduced umbilical cord-derived mesenchymal stem cells (MSC-Zk8) were cocultured with the tendon cells (D).

The expression of Egr-2 was significantly decreased when inflammatory response was induced in the tendon cells (B). When the Zkscan8-introduced umbilical cord-derived mesenchymal stem cells (MSC-Zk8) were cocultured with the tendon cells (D), the expression of Egr-2 was increased significantly and was recovered to a level comparable to that of the normal tendon cells (A).

From these results, it can be seen that, by coculturing tendon cells with the Zkscan8-introduced umbilical cord-derived mesenchymal stem cells (MSC-Zk8) under an inflammatory environment, the expression of inflammation-mediating factors can be decreased and the expression of the inflammation-inhibiting factor IL-10 can be increased. In addition, the expression of MMP-3 was decreased, the expression of the growth factors bFGF, TGFβ2, HGF and bFGF was increased, and the expression of Egr-1 and Egr-2 was increased. Therefore, it can be seen that inflammatory response can be suppressed, inhibited, alleviated or recovered by overexpressing the Zkscan8 gene or coculturing with the Zkscan8-introduced umbilical cord-derived mesenchymal stem cells (MSC-Zk8).

### Analysis of expression pattern of fat, bone, cartilage, tendon, muscle and nerve-related markers in Zkscan8-introduced umbilical cord-derived mesenchymal stem cells (MSC-Zk8)

Tendon tissue has many similarities to bone, cartilage and muscle tissues. Therefore, it was investigated whether Zkscan8 also affects the markers of fat, bone, cartilage, tendon, muscle and nerve that constitute the musculoskeletal system, in addition to tendon tissue. Specifically, Zkscan8-introduced umbilical cord-derived mesenchymal stem cells (MSC-Zk8) were prepared and the expression pattern of various genes including musculoskeletal markers was analyzed 2 and 3 days later.

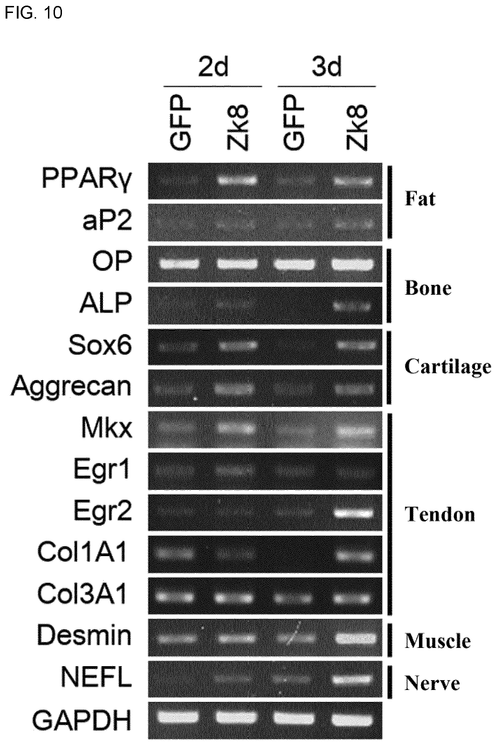

FIG. 10 shows a result of analyzing the expression pattern of fat, bone, cartilage, tendon, muscle and nerve cell-related markers in the Zkscan8-introduced umbilical cord-derived mesenchymal stem cells (MSC-Zk8) and the control group (MSC-GFP). The expression of the fat, bone, cartilage, tendon, muscle and nerve-related genes was increased on the whole in the Zkscan8-introduced umbilical cord-derived mesenchymal stem cells (MSC-Zk8). In particular, the expression of ALP (alkaline phosphatase), Sox6 (SRY-box transcription factor 6), Egr-2, Col1A1, desmin and NEFL (neurofilament light polypeptide) in the Zkscan8-introduced umbilical cord-derived mesenchymal stem cells (MSC-Zk8) was remarkably increased on day 3 as compared to the control group (MSC-GFP). This suggests that the overexpression of Zkscan8 affects the musculoskeletal system including bone, cartilage, muscle and nerve.

### Macroscopic evaluation

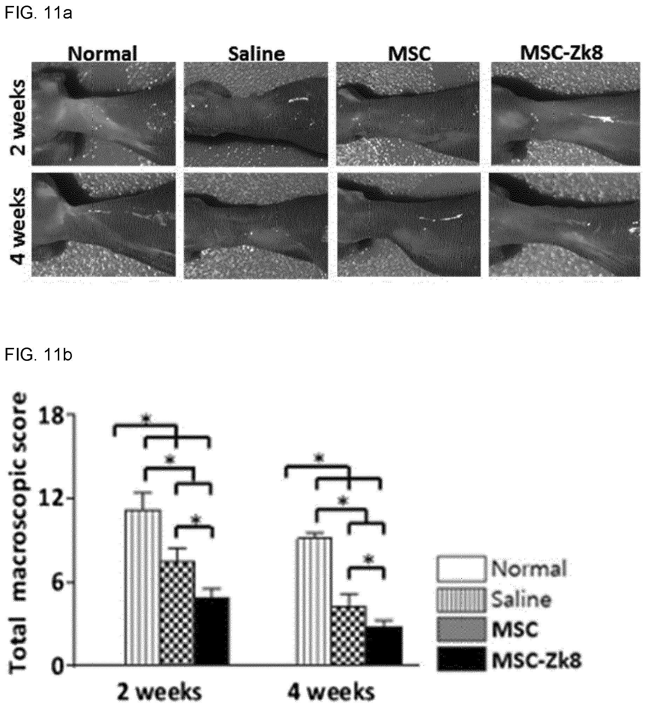

FIG. 11a shows the macroscopic images of the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. The tissues around the defective region were removed for easier observation of the defect state of the tendon.

FIG. 11b shows a result of analyzing total macroscopic score for the supraspinatus tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment.

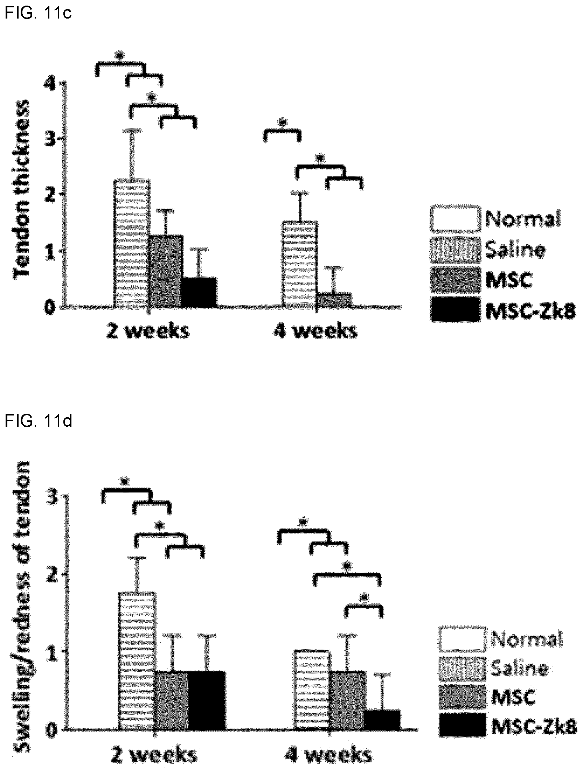

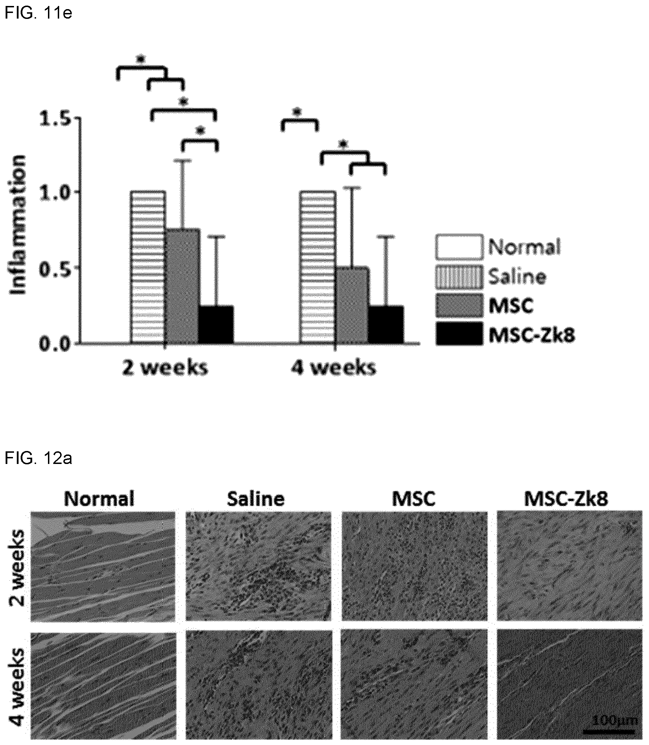

FIG. 11c shows a result of analyzing tendon thickness for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment, FIG. 11d shows a result of analyzing swelling/redness of tendon for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment, and FIG. 11e shows a result of analyzing inflammation for the supraspinatus tendon of a normal group (Normal), a physiological saline group (Saline), an umbilical cord-derived mesenchymal stem cell group (MSC) and a Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

As shown in FIG. 11a and FIG. 11b, the total macroscopic score, which is a measure of apparently severe injury, was compared between the groups. At week 2, the total macroscopic score was 4.88 ± 0.64 for the Zkscan8-introduced umbilical cord-derived mesenchymal stem cell group (MSC-Zk8), but 11.13 ± 1.25 (p < 0.000) and 7.38 ± 1.06 (p < 0.000) for the physiological saline group and the umbilical cord-derived mesenchymal stem cell group, respectively, indicating severe injury. That is to say, it can be seen that the damage of tendon cells was significantly lower in the Zkscan8-introduced umbilical cord-derived mesenchymal stem cell group (MSC-Zk8) as compared to other groups.

At week 4, the total macroscopic score of the Zkscan8-introduced umbilical cord-derived mesenchymal stem cell group (MSC-Zk8) was 2.75 ± 0.46. In contrast, the total macroscopic score was 9.13 ± 0.35 (p < 0.000) and 4.25 ± 0.89 (p < 0.000), respectively, for the physiological saline group and the umbilical cord-derived mesenchymal stem cell group, indicating remarkably sever injury as compared to MSC-Zk8. From these experiments, it can be seen that the overexpression of Zkscan8 significantly increases the ability of recovering from tissue injury the umbilical cord-derived mesenchymal stem cells.

To specifically describe the tendon thickness, which is a parameter related with inflammation, the tendon thickness was 0.50 ± 0.53 for the Zkscan8-introduced umbilical cord-derived mesenchymal stem cell group (MSC-Zk8), and 2.25 ± 0.89 (p < 0.000) and 1.25 ± 0.46 for the physiological saline group and the umbilical cord-derived mesenchymal stem cell group, respectively. That is to say, the tendon thickness of the Zkscan8-introduced umbilical cord-derived mesenchymal stem cell group (MSC-Zk8) was significantly smaller as compared to other groups. Accordingly, it can be seen that the tendon thickness of the Zkscan8-introduced umbilical cord-derived mesenchymal stem cell group (MSC-Zk8) was recovered significantly as inflammatory response in the tendon was inhibited.

At week 4, the tendon thickness was 0.00 ± 0.00 for the Zkscan8-introduced umbilical cord-derived mesenchymal stem cell group (MSC-Zk8), and 1.50 ± 0.53 (p < 0.000), 0.25 ± 0.46 for the physiological saline group and the umbilical cord-derived mesenchymal stem cell group, respectively. That is to say, the tendon thickness was still the smallest for the Zkscan8-introduced umbilical cord-derived mesenchymal stem cell group (MSC-Zk8).

To specifically describe the swelling/redness of the tendon, which is a parameter related with inflammation, the swelling/redness at week 2 was 0.75 ± 0.46 for the Zkscan8-introduced umbilical cord-derived mesenchymal stem cell group (MSC-Zk8), and 1.75 ± 0.46 (p < 0.000) and 0.75 ± 0.46 for the physiological saline group and the umbilical cord-derived mesenchymal stem cell group, respectively. The level of tendon swelling/redness of the Zkscan8-introduced umbilical cord-derived mesenchymal stem cell group (MSC-Zk8) was significantly lower as compared to other groups. That is to say, it can be seen that the swelling/redness of tendon was recovered in the Zkscan8-introduced umbilical cord-derived mesenchymal stem cell group (MSC-Zk8).

At week 4, the swelling/redness of the tendon was 0.25 ± 0.46 for the Zkscan8-introduced umbilical cord-derived mesenchymal stem cell group (MSC-Zk8), and 1.00 ± 0.00 (p = 0.001) and 0.75 ± 0.46 (p = 0.029) for the physiological saline group and the umbilical cord-derived mesenchymal stem cell group, respectively. That is to say, the level of tendon swelling/redness was still the lowest in the Zkscan8-introduced umbilical cord-derived mesenchymal stem cell group (MSC-Zk8). Accordingly, it can be seen that the swelling/redness of tendon was recovered remarkably over time in the Zkscan8-introduced umbilical cord-derived mesenchymal stem cell group (MSC-Zk8).

To specifically describe the inflammation of the tendon, which is a parameter related with inflammation, the inflammation of the tendon at week 2 was 0.25 ± 0.46 for the Zkscan8-introduced umbilical cord-derived mesenchymal stem cell group (MSC-Zk8), and 1.00 ± 0.00 (p = 0.001) and 0.75 ± 0.46 (p = 0.029) for the physiological saline group and the umbilical cord-derived mesenchymal stem cell group, respectively. The inflammation level of the tendon was significantly lower in the Zkscan8-introduced umbilical cord-derived mesenchymal stem cell group (MSC-Zk8) as compared to other groups. That is to say, it can be seen that the inflammation of the tendon was recovered in the Zkscan8-introduced umbilical cord-derived mesenchymal stem cell group (MSC-Zk8).

At week 4, the inflammation level of the tendon was 0.25 ± 0.46 for the Zkscan8-introduced umbilical cord-derived mesenchymal stem cell group (MSC-Zk8), and 1.00 ± 0.00 (p = 0.001) and 0.50 ± 0.53 for the physiological saline group and the umbilical cord-derived mesenchymal stem cell group, respectively. The inflammation level of the tendon was still the lowest in the Zkscan8-introduced umbilical cord-derived mesenchymal stem cell group (MSC-Zk8). That is to say, it can be seen that the inflammation of the tendon was recovered remarkably over time in the Zkscan8-introduced umbilical cord-derived mesenchymal stem cell group (MSC-Zk8).

When an inflammatory disease occurs in the rotator cuff as a result of rotator cuff injury, swelling, redness and thickening occur due to the inflammation. As a result, the patient experiences limitation in motion and severe pain. However, because the administration of the Zkscan8-introduced umbilical cord-derived mesenchymal stem cell group (MSC-Zk8) prevents or recovers the thickening, swelling, redness, inflammation, etc. of the rotator cuff, the pain and symptoms of the patient that can be caused by the inflammatory disease of the tendon can be alleviated, prevented or treated.

### Histological evaluation

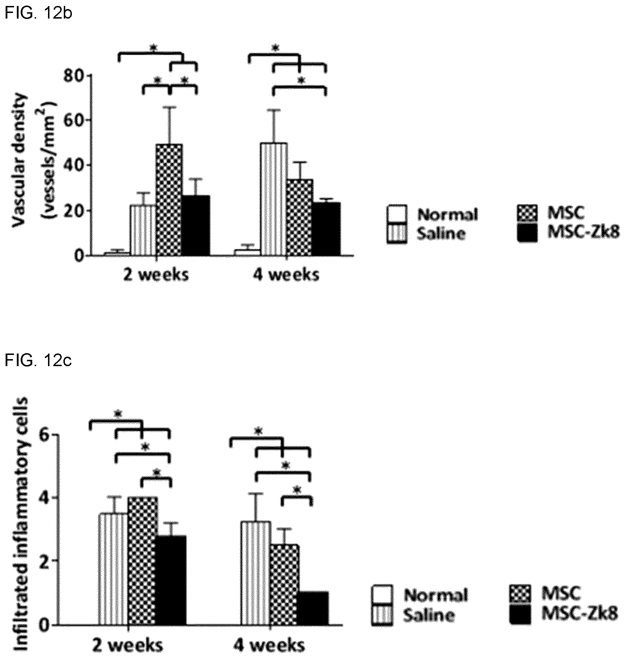

FIG. 12a shows the H&E staining images of the supraspinatus tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment (magnification: x100), FIG. 12b shows a result of analyzing vascular density for the supraspinatus tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment, and FIG. 12c shows a result of analyzing infiltrated inflammatory cells for the supraspinatus tendon of the normal group (Normal), the physiological saline group (Saline), the umbilical cord-derived mesenchymal stem cell group (MSC) and the Zkscan8-introduced umbilical cord-derived mesenchymal stem cell group (MSC-Zk8) at weeks 2 and 4 after the beginning of experiment. All quantitative data are presented as mean ± standard error (S.E.M) (*p < 0.05).

As shown in FIG. 12a and FIG. 12b, the vascular density at week 2 after the beginning of experiment was 49.22 ± 16.60 for the umbilical cord-derived mesenchymal stem cell group (MSC-GFP), 22.14 ± 5.70 (p = 0.033) for the physiological saline group, and 26.34 ± 7.54 (p = 0.004) for the Zkscan8-introduced umbilical cord-derived mesenchymal stem cell group (MSC-Zk8). That is to say, it can be seen that nonspecific vascularization occurred a lot in the umbilical cord-derived mesenchymal stem cell group (MSC-GFP).

At week 4 after the beginning of experiment, the vascular density was 22.94 ± 2.26 for the Zkscan8-introduced umbilical cord-derived mesenchymal stem cell group (MSC-Zk8) and 49.95 ± 14.59 (p = 0.004) for the physiological saline group. That is to say, it can be seen that nonspecific vascularization occurred significantly less in the Zkscan8-introduced umbilical cord-derived mesenchymal stem cell group (MSC-Zk8) as compared to the umbilical cord-derived mesenchymal stem cell group (MSC-GFP).

As shown in FIG. 12a and FIG. 12c, the degree of infiltration of inflammatory cells at week 2 after the beginning of experiment was 2.75 ± 0.46 for the Zkscan8-introduced umbilical cord-derived mesenchymal stem cell group (MSC-Zk8), and 3.50 ± 0.53 (p = 0.001) and 4.00 ± 0.00 (p < 0.001) for the physiological saline group and the umbilical cord-derived mesenchymal stem cell group, respectively. That is to say, it can be seen that the degree of infiltration of inflammatory cells was significantly lower in the umbilical cord-derived mesenchymal stem cell group (MSC-GFP) as compared to other groups.

At week 4 after the beginning of experiment, the degree of infiltration of inflammatory cells was 1.00 ± 0.00 for the Zkscan8-introduced umbilical cord-derived mesenchymal stem cell group (MSC-Zk8), 3.25 ± 0.89 (p < 0.001) for the physiological saline group, and 2.50 ± 0.53 (p = 0.001) for the umbilical cord-derived mesenchymal stem cell group. That is to say, it can be seen that the degree of infiltration of inflammatory cells was significantly decreased in the Zkscan8-introduced umbilical cord-derived mesenchymal stem cell group (MSC-Zk8) as compared to other groups.

When mesenchymal stem cells are used for therapeutic purposes, they can be the targets of innate and acquired immune responses by NK cells, T-cells and B-cells. These two mechanisms in response to foreign substances exist in all people and cannot be avoided for both heteroplantation and homoplantation. In the present disclosure, genes related with inflammatory diseases, particularly musculoskeletal inflammatory diseases, were identified and the effect of their overexpression was investigated. In particular, as a result of using human umbilical cord-derived mesenchymal stem cells for overexpression of the Zkscan8 gene, it was confirmed that, even though the cells were injected into rats, the tendon damaged by inflammation was regenerated and recovered with no side effect such as immune rejection.

In an example of the present disclosure, Zkscan8-introduced umbilical cord-derived mesenchymal stem cells were prepared for overexpression of Zkscan8. When the cells were treated, vascularization and inflammation were decreased significantly as compared to when only umbilical cord-derived mesenchymal stem cells were treated, and the thickness, swelling/redness and inflammation of the tendon were also decreased remarkably. In addition, when the Zkscan8-introduced umbilical cord-derived mesenchymal stem cells were treated, inflammation could be controlled significantly and powerfully as the expression of the anti-inflammatory cytokine IL-10 was increased.

In conclusion, the Zkscan8-introduced umbilical cord-derived mesenchymal stem cells may be used clinically to suppress and alleviate the inflammatory response of tendon tissue by regulating the immune response increased by inflammation, without inducing immune rejection.

## Claims

1. A composition for preventing or treating an inflammatory disease, comprising a Zkscan8 (zinc finger protein with KRAB and SCAN domains 8) protein as an active ingredient.

2. The composition according to claim 1, wherein the Zkscan8 is represented by an amino acid sequence of SEQ ID NO 2.

3. The composition according to claim 1, wherein the inflammatory disease is one or more selected from a group consisting of dermatitis, allergy, atopy, asthma, bronchitis, conjunctivitis, periodontitis, rhinitis, otitis media, sore throat, tonsillitis, pneumonia, gastric ulcer, gastritis, Crohn's disease, colitis, peritonitis, osteomyelitis, meningitis, encephalitis, cystic fibrosis, stroke, hemorrhoids, gout, ankylosing spondylitis, spondyloarthropathy, enteropathy, spondylitis, rheumatic fever, lupus, fibromyalgia, psoriatic arthritis, osteoarthritis, rheumatoid arthritis, frozen shoulder, tendinitis, tenosynovitis, peritendinitis, tenosynovitis, myositis, hepatitis, cystitis, nephritis, Sjogren's syndrome, multiple sclerosis and acute and chronic inflammatory diseases.

4. The composition according to claim 1, wherein the inflammatory disease is one or more selected from a group consisting of psoriatic arthritis, osteoarthritis, rheumatoid arthritis, frozen shoulder, tendinitis, tenosynovitis, peritendinitis, tenosynovitis and myositis.

5. A pharmaceutical composition for preventing or treating an inflammatory disease, comprising a Zkscan8 (zinc finger protein with KRAB and SCAN domains 8) gene, a vector comprising the gene, a cell comprising the vector or a culture thereof as an active ingredient.

6. The composition according to claim 5, wherein the vector is a linear DNA, a plasmid DNA or a recombinant viral vector.

7. The composition according to claim 5, wherein the recombinant virus is any one selected from a group consisting of retrovirus, adenovirus, adeno-associated virus, herpes simplex virus and lentivirus.

8. The composition according to claim 5, wherein the cell is one or more selected from a group consisting of a stem cell, a dendritic cell, an autologous tumor cell and an established tumor cell.

9. The composition according to claim 8, wherein the stem cell is a mesenchymal stem cell.

10. The composition according to claim 9, wherein the mesenchymal stem cell is an umbilical cord-derived mesenchymal stem cell.

11. The composition according to claim 5, wherein the composition inhibits the expression or activity of COX-2, mPGES-1, TNF-α, MMP-1 and MMP-3, and increases the expression of IL-10.

12. A method for screening a composition for preventing or treating an inflammatory disease, comprising:
(a) a step of contacting cells treated with a stimulation that can induce an inflammatory response with a test substance; and
(b) a step of measuring the expression level of Zkscan8 (zinc finger protein with KRAB and SCAN domains 8) in the sample,
wherein, if the expression level of the Zkscan8 is increased as compared to a normal control group in the step (b), the test substance is determined as a composition for preventing or treating an inflammatory disease.

13. The method according to claim 12, wherein the inflammatory disease is one or more selected from a group consisting of psoriatic arthritis, osteoarthritis, rheumatoid arthritis, frozen shoulder, tendinitis, tenosynovitis, peritendinitis, tenosynovitis and myositis.
